# EUROPEAN PATENT SPECIFICATION

(11) **EP 2 619 306 B1**
(45) Date of publication and mention of the grant of the patent: **20.05.2015**
(21) Application number: 11758244.5
(22) Date of filing: 22.09.2011
(51) Int. Cl.: C12N 15/10, C12N 15/62, C12N 9/02, C12P 21/02

(54) **SURFACE DISPLAY OF POLYPEPTIDES CONTAINING A METAL PORPHYRIN OR A FLAVIN**
ZELLOBERFLÄCHENPRÄSENTATION VON METALL-POPHYRIN ODER -FLAVIN ENTHALTENEN POLYPEPTIDEN
PRÉSENTATION DE POLYPEPTIDES CONTENANT UNE PORPHYRINE MÉTALLIQUE OU UNE FLAVINE À LA SURFACE D'UNE CELLULE HÔTE

(30) Priority: 23.09.2010 EP 10179060
(43) Date of publication of application: 31.07.2013
(73) Proprietor: Autodisplay Biotech GmbH, 40225 Düsseldorf (DE)
(72) Inventor: SCHUMACHER, Stephanie, 40223 Düsseldorf (DE); BERNHARDT, Rita, 66129 Saarbrücken (DE); HANNEMANN, Frank, 66125 Saarbrücken (DE); JOSE, Joachim, 40589 Düsseldorf (DE)
(74) Representative: Weickmann & Weickmann
(86) International application number: PCT/EP2011/066517
(87) International publication number: WO 2012/038508

(56) References cited:
- WO-A1-97/35022
- WO-A2-02/070645
- WO-A2-2004/088307
- WO-A2-2005/086826
- JOSE J ET AL: "Functional display of active bovine adrenoxin on the surface of E. Coli by chemical incorporation of the 2Fe-2S cluster", CHEMBIOCHEM - A EUROPEAN JOURNAL OF CHEMICAL BIOLOGY, WILEY VCH, WEINHEIM, DE, vol. 2, 1 January 2001 (2001-01-01), pages 695-701, XP002231689, ISSN: 1439-4227, DOI: 10.1002/1439-7633(20010903)2:9<695::AID-CB IC695>3.0.CO;2-S
- TATSUMI RYOKO ET AL: "TolC-dependent exclusion of porphyrins in Escherichia coli.", JOURNAL OF BACTERIOLOGY SEP 2008 LNKD- PUBMED:18641137, vol. 190, no. 18, September 2008 (2008-09) , pages 6228-6233, XP002664991, ISSN: 1098-5530 cited in the application
- JOSE J ET AL: "Cellular surface display of dimeric Adx and whole cell P450-mediated steroid synthesis on E. coli", JOURNAL OF BIOTECHNOLOGY, ELSEVIER SCIENCE PUBLISHERS, AMSTERDAM, NL, vol. 95, no. 3, 23 May 2002 (2002-05-23), pages 257-268, XP002296795, ISSN: 0168-1656, DOI: 10.1016/S0168-1656(02)00030-5 cited in the application
- JOACHIM JOSE: "Autodisplay: efficient bacterial surface display of recombinant proteins", APPLIED MICROBIOLOGY AND BIOTECHNOLOGY, SPRINGER, BERLIN, DE, vol. 69, no. 6, 1 February 2006 (2006-02-01), pages 607-614, XP019332065, ISSN: 1432-0614, DOI: 10.1007/S00253-005-0227-Z cited in the application
- JOSE J ET AL: "The autodisplay story, from discovery to biotechnical and biomedical applications", MICROBIOLOGY AND MOLECULAR BIOLOGY REVIEWS, AMERICAN SOCIETY FOR MICROBIOLOGY, US, vol. 71, no. 4, 1 December 2007 (2007-12-01), pages 600-619, XP002577399, ISSN: 1092-2172, DOI: 10.1128/MMBR.00011-07 cited in the application
- SCHOPFER C R ET AL: "Identification of elicitor-induced cytochrome P450s of soybean (Glycine max L.) using differential display of mRNA.", MOLECULAR & GENERAL GENETICS : MGG MAY 1998 LNKD- PUBMED:9648734, vol. 258, no. 4, May 1998 (1998-05), pages 315-322, XP002664992, ISSN: 0026-8925

## Description

The present invention relates to a method for functionally displaying a recombinant polypeptide containing a prosthetic group on the surface of a host cell, wherein the prosthetic group is selected from metal porphyrins and flavins.

Over the past 30 years, it has become clear that enzymes hold great potential for industry. They are most remarkable biomolecules because of their extraordinary specificity and catalytic power [1]. The specificity and (enantio- and regio-)selectivity of certain enzymatic transformations makes them attractive for the production of fine chemicals and pharmaceutical intermediates. To date, more than 500 products are manufactured by enzymes. Well-known examples are ephedrine, aspartame and amoxicillin [2,3,4].

Cytochromes P450 enzymes have been discovered about 50 years ago and are ubiquitously distributed enzymes, which possess high complexity and display a broad field of catalytic activities. They are hemoproteins, which means, they contain a porphyrin ring system. The P450 enzyme family is involved in the biotransformation of drugs, the bioconversion of xenobiotics, the metabolism of chemical carcinogens, the biosynthesis of physiologically important compounds such as steroids, fatty acids, eicosanoids, fat-soluble vitamins, bile acids, the conversion of alkanes, terpenes, and aromatic compounds as well as the degradation of herbicides and insecticides [5]. Furthermore, there is a broad versatility of reactions catalysed by cytochromes P450, such as carbon hydroxylation, heteroatom oxygenation, dealkylation, epoxidation, aromatic hydroxylation, reduction, and dehalogenation.

Despite their very interesting features for industrial applications, the use of P450 enzymes for wide biotechnology needs is still limited, due to their difficulty in handling. With the exception of a few bacterial P450s, the vast majority needs a certain membrane contact or environment to fold into an active form. Within those membrane associated P450s, two classes can be identified: a mitochondrial and a microsomal. At the moment there are two different ways to use these enzymes for synthetic purposes. They are either purified after recombinant expression and reconstituted with an artificial membrane system, or they are expressed and used in a whole cell context. Both ways have their limitations. Reconstituted membrane vesicles with P450 enzymes are laborious to produce and they are absolutely not suited for industrial applications. Using whole cells with intrinsic P450s limits the set of substrates to be converted to those which are able to cross membranes [6].

Among other systems for the secretion of proteins in Gram-negative bacteria, the autotransporter pathway represents a solution of impressing simplicity. It is possible to transport a protein, regardless whether it is recombinant or the natural passenger, to the actual outer membrane, as long as its coding region lies between a typical signal peptide and a C-terminal domain called β-barrel. Based on these findings the autodisplay system has been developed by the use of the natural *E*. *coli* autotransporter protein AIDA-I (the adhesin involved in diffuse adherence) in an *E*. *coli* host background [9]. Autodisplay has been used for the surface display of random peptide libraries that were successfully screened for the identification of new enzyme inhibitors, and the display of functional enzymes like esterases, oxidoreductases and electron transfer proteins [10].

During the autodisplay of bovine adrenodoxine, which serves as an electron donor for mitochondrial p450s, two major observation were made [11,12]. First, it could be shown, that it is possible to incorporate an inorganic, prosthetic group into an apoprotein expressed by autodisplay at the cell surface by a simple titration step to yield a functional electron donor without loss of cell viability or cell integrity. And second, after external addition of the purified P450s CYP11B1 and CYP11A1, a functional whole cell biocatalyst was obtained for efficient synthesis of different steroids. Therefore the aim of the present invention is, to investigate, whether it is possible to autodisplay a P450 enzyme in a functional form on the surface of *E*. *coli.* This could provide a new expression platform for the highly interesting group of P450 enzymes with the perspective of being applicable in industrial processes.

### Background

Cytochromes P450 are external monooxygenases. Monooxygenases (mixed function oxidases) catalyse the incorporation of a single atom of molecular oxygen into a substrate with the concomitant reduction of the other atom to water. Monooxygenases are divided into two classes: internal and external. Internal monooxygenases extract two reducing equivalents from the substrate to reduce one atom of dioxygen to water, whereas external monooxygenases utilize an external reductant. While initially the microsomal drug and xenobiotic-metabolising enzymes were referred to as mixed function oxidases, in more recent years the term monooxygenase became the widely accepted one.

Cytochromes P450 got their name from their character as hemoproteins as well as their unusual spectral properties displaying a typical absorption maximum of the reduced CO-bound complex at 450 nm: cytochrome stands for a hemoprotein, P for pigment and 450 reflects the absorption peak of the CO complex at 450 nm. The ability of reduced P450 to produce an absorption peak at 450 nm upon CO binding is still used for the estimation of the P450 content (Omura and Sato, 1964). The red shift of about 30 nm as observed in cytochromes P450 means that the distribution of electron density at the heme is significantly perturbed as compared to other cytochromes. It has been documented that the cause of this is the thiolate sulphur, which by means of a direct bond to the iron causes this effect. The Soret band (named after its discoverer) describes the absorption band of hemoproteins at about 380-420 nm.

Cytochrome P450 systems mainly catalyse the following reaction:

RH + O₂ +NAD(P)H + H⁺ → ROH + H₂O + NAD(P)⁺

They are involved in reactions as diverse as e.g. hydroxylation, N-, O- and S-dealkylation, sulphoxidation, epoxidation, deamination, desulphuration, dehalogenation, peroxidation, and N-oxide reduction. This diversity of catalysed reactions and, of course, the high amount of acceptable substrates is attractive for biotechnological application in particular when it can be transferred to industrial needs.

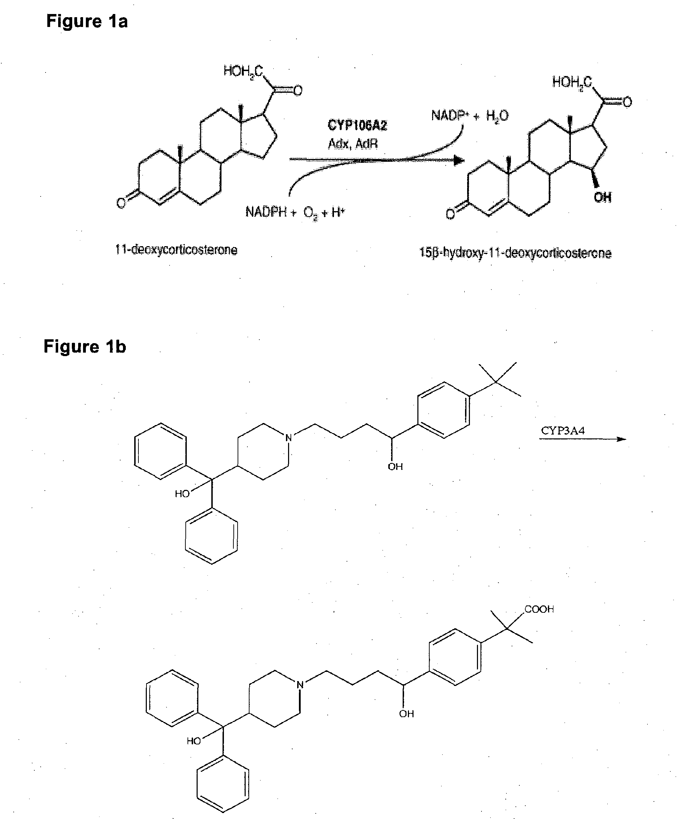

CYP106A2 is a bacterial steroid hydroxylase from *Bacillus megaterium* ATCC 13368. Since it is soluble and easy to express it has application for biotechnological purposes. Recently, it was possible to design a whole cell bioconversion system for steroids using a mixed system composed of the bovine mitochondrial electron transfer system AdR and Adx and the bacterial enzyme CYP106A2. This mixed P450 monooxygenase system was expressed in *E*. *coli* cells. Those successful experiments opened the door to facilitate the application of molecular evolution approaches in order to select mutants of the cytochrome with higher stability, activity, and changed regio-and stereo-specificity suitable to produce hydroxylated steroid derivatives using a biological transformation process [6]. CYP106A2 catalyzes as main reaction route the 15β-hydroxylation of several steroids, e.g. 11-deoxycorticosterone, testosterone, progesterone, and corticosterone (Fig. 1a) [7]. One disadvantage of this system is the limitation for substrates which are membrane permeable like corticosteroids. A successful expression via autodisplay could broaden the variety of substrates and therefore make the P450 monooxygenase system more valuable for future research.

CYP3A4 is the quantitative most important CYP- enzyme and involved in the oxidation of the largest range of substrates of all CYPs. In humans it is predominantly found in the liver and often allows prodrugs to be activated and absorbed. Inhibition or induction of CYP3A4 is a major problem in the daily clinical routine, since it often causes drug-drug interactions or side effects. Induction can lead to the fast inactivation of the applied drug and in consequence to plasma levels so low, that they do not have the desired therapeutic effect anymore. A commonly used CYP3A4 inductor is the anticonvulsant Carbamazepin. Inhibition instead can cause major intoxications due to plasma levels far beyond the therapeutic dose. On the other hand the capability of inhibition is used in the antiretroviral therapy to lower side effects and make it more bearable for patients. Ritonavir is given in a subtherapeutic dose to inhibit the enzyme and booster the effect of further antiretroviral drugs such as Lopinavir. A well-documented example is that of terfenadine, a nonsedating antihistamine (Fig. 1 b). The oxidation of terfenadine is catalyzed very rapidly by CYP3A4 to its major metabolite fexofenadine which is responsible for the pharmacological activity. [8]

The *E*. *coli* outer membrane channel-tunnel protein TolC is involved in the exclusion of harmful substances such as antibiotics, dyes, organic solvents, and detergents. The crystal structure of the TolC protein recently has been determined. The TolC protein is composed of a transmembrane domain and a periplasmic domain and forms a homotrimer. The periplasmic barrel structure of TolC is connected to drug efflux pump proteins such as AcrB and AcrE, which are located on the inner membrane. Clamp proteins such as AcrA and ArcF link TolC and pump proteins in the periplasmic space. Pump proteins seem to transport toxic cytoplasmic or periplasmic substances into the extracellular space across the outer membrane via the TolC channel [26].

Porphyrins can act as photosensitizers. If porphyrins accumulate, they can be toxic, as the cells can become sensitive to near-UV irradiation. TolC is involved in porphyrin transport across the cell membrane and provides a mechanism to eliminate superfluous or/and toxic porphyrins. The TolC outer membrane channel-tunnel protein can function together with inner membrane efflux pump proteins. Therefore, an inner membrane pump(s) or exporter(s) is assumed to be involved in porphyrin exclusion in combination with TolC. Porphyrin(ogen) exclusion is considered as a two-step process. In this process, porphyrin(ogen)s are transported to the periplasm by a TolC-independent mechanism and then are transported across the outer membrane by the TolC-dependent efflux system [26].

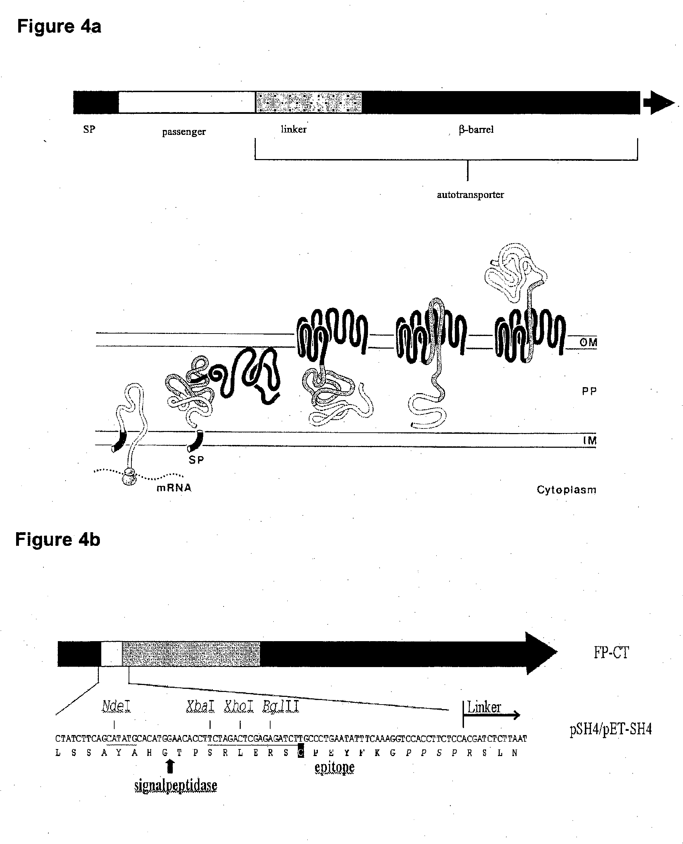

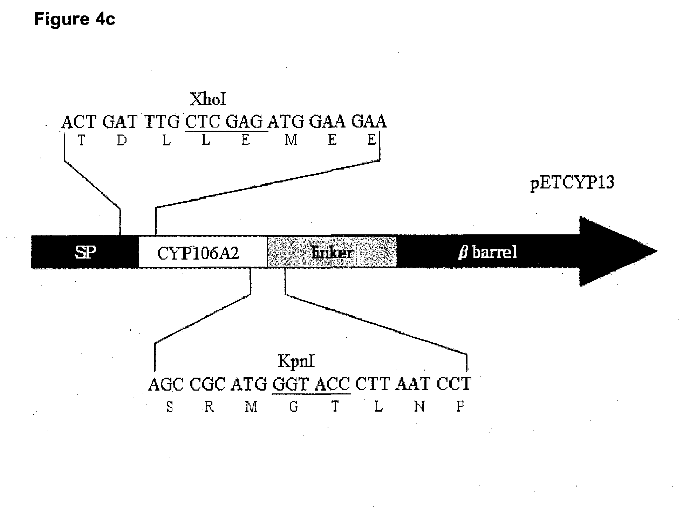

Autodisplay is based on the secretion mechanism of the autotransporter family or proteins [13]. A concept for this secretion mechanism was proposed concurrently with the first autotranspoter protein, IgA1protease from *Neisseria gonorrhoeae* (Fig. 4a) [14]. With the aid of a typical signal peptide, the precursor is transported across the inner membrane. Arrived in the periplasm, the C terminal part of the precursor forms a porin-like structure, a so-called β-barrel, within the outer membrane and through this pore the N terminally attached passenger (the actual protease) is translocated to the cell surface. To obtain full surface exposure of the passenger, a linker peptide is required in between the β-barrel and the passenger.

For the development of the autodisplay system the β-barrel and the linker region of AIDA-I were combined in frame with the signal peptide of the cholera toxin β-subunit (CTB) and a strong constitutive promoter (P_{TK}) within a medium copy number plasmid backbone [15]. Into the linker regions used for autodisplay, protease cleavage sites for the sequence specific release of the passenger protein, as well as epitopes for detection by monoclonal antibodies were inserted. An antibody independent detection method, which requires only the addition of a single cysteine in the linker region, was developed for autodisplay and was named "Cystope tagging" [16,17]. A schematic description of the structure of a typical artificial autotransporter protein used for autodisplay is given in Fig. 4b. As mentioned above, the terminal step in autodisplay requires the translocation of the passenger through a size-limited pore formed by the β-barrel. This means that the passenger is not allowed to acquire a stable three dimensional conformation during transport to maintain a transport compatible state [18,19]. In case of stable folding, transport is blocked in the periplasm [19]. As a wide variety of passenger proteins with high biotechnological impact contain disulfide bridges and these bonds are normally formed in the periplasm of *E*. *coli,* a DsbA-negative mutant strain of *E*. *coli* (JK321) was constructed and shown to facilitate the autodisplay of such types of proteins as well [19]. In summary, the autodisplay system consists of vectors encoding various artificial autotransporter genes using the β-barrel from AIDA-I and different parts of its linkerregion. Dependent on the application, different modifications of the linker regions, various signal peptides under the control of inducible or constitutive promoters, mutant strains of *E*. *coli* supporting the transport and the surface display by the autotransporter pathway and detection methods are now available, that allow to follow surface translocation, preferentially independent of the protein domain used as a passenger. It is obvious, that autodisplay is restricted to Gram-negative bacteria i.e. *E*. *coli* or *Salmonella* as host organisms. Beyond this limitation, the autodisplay system has interesting activa. First, more than 100.000 active enzyme molecules can be displayed per single cell of *E*. *coli* without loss in cell integrity. Second, dimers or multimers can be formed spontaneously at the cell surface by subunits expressed from monomeric genes, which is a unique feature of this surface display system and due to the free motility of the anchoring motif, the β-barrel within the outer membrane. Third, EP 02718168 describes that anorganic prosthetic groups (e.g. 2Fe-2S) can be incorporated by a single step/one vial procedure without affecting cell viability, another feature that has not been described for any other surface display system so far. These features have been used in combination for the construction of whole cell biocatalysts displaying functional enzymes which were used as technological tools for the regio- and enantioselective synthesis of products, especially from substrates with several identical reactive groups, including sugars polyalcohols and steroids with high efficiency [12,21,22].

EP 02718168 describes autodisplay of adrenodoxin on an *E*. *coli* cell. Adrenodoxin belongs to the [2Fe-2S] ferredoxins, a family of small acidic iron-sulfur proteins. When displayed on the surface, the adrenodoxin is present in a non-functional form, because no prosthetic group is present. According to EP 02718168, a functional adrenodoxin attached to the cell surface can be obtained by contacting the adrenodoxin molecule with an exogenous [2Fe-2S] cluster serving as a prosthetic group.

The problem of the present invention is the provision of surface displayed enzymes comprising metal porphyrin-containing or flavin containing prosthetic groups. It was surprisingly found that by recombinant expression of these enzymes by surface display on a Gram-negative bacterium, a functional enzyme comprising the metal porphyrin-containing or flavin containing prosthetic group could be identified on the cell surface without introducing an exogenous prosthetic group, as described for enzymes containing [2Fe-2S] clusters in WO 02/070645. In other words, polypeptides comprising prosthetic groups containing a metal porphyrin or a flavin can translocate to the cell surface in a conformation capable of retaining the prosthetic group when crossing the outer membrane, for example by mediation of the omp85 pathway. The prosthetic group may also be transported to the cell surface independently from the surface-displayed enzyme. Mechanisms are known for elimination of superfluous or toxic compounds, including compounds suitable as prosthetic groups (e.g., porphyrins), from the cell. In the present invention, it has been surprisingly found that metal porphyrins transported across the cell membrane into the extracellular space independently from the enzyme/autotransporter construct can contact the enzyme displayed on the cell surface to a form an active enzyme (see Example 2).

Thus, a first aspect of the present invention is a method for displaying a recombinant polypeptide containing a prosthetic group on the surface of a host cell, wherein the prosthetic group comprises a metal porphyrin or a flavin, said method comprising the steps:
(a) providing a host cell transformed with a nucleic acid fusion operatively linked with an expression control sequence said nucleic acid fusion comprising:
   (i) a portion encoding a signal peptide,
   (ii) a portion encoding the recombinant polypeptide to be displayed,
   (iii) a portion encoding a transmembrane linker, and
   (iv) a portion encoding the transporter domain of an autotransporter,
      and
(b) culturing the host cell under conditions wherein the nucleic acid fusion is expressed and the expression product comprising the recombinant polypeptide containing the prosthetic group is displayed on the surface of the host cell,
wherein the prosthetic group is transported to the cell surface independently from the expression product comprising the recombinant polypeptide. Optionally, the nucleic acid fusion comprises a portion encoding a protease recognition site. By the method of the present invention, a functional recombinant polypeptide can be displayed. As indicated above, display of the functional recombinant polypeptide of the present invention comprising a prosthetic group containing a metal porphyrin or a flavin does not require an exogenously added prosthetic group. In the present invention, the prosthetic group can be produced by the host cell ("endogenously produced prosthetic group"). In a preferred embodiment, the method of the present invention, in particular step (b), is performed with the proviso that the surface-displayed recombinant polypeptide is not contacted with an exogenous prosthetic group being a metal porphyrin or a flavin. In this context, "exogenous prosthetic group" refers to a prosthetic group not produced by the host cell.

The recombinant polypeptide to be displayed may also be termed "passenger", "passenger polypeptide" or "passenger protein".

Step (a) of the methods of the present invention refers to the provision of a host cell. The host cell used in the method of the present invention is preferably a bacterium, more preferably a Gram-negative bacterium, particularly an enterobacterium such as *E*. *coli.*

According to the present invention, a host cell, particularly a host bacterium is provided which is transformed with a nucleic acid fusion operatively linked with an expression control sequence, i.e. a promoter, and optionally further sequences required for gene expression in the respective host cell. The skilled person knows suitable promoters and expression control sequences. The promoter or/and the expression control sequence may be homologous or heterologous to the host cell. Preferably, the nucleic acid fusion is located on a recombinant vector, e.g. a plasmid vector. The host cell may be transformed with at least one nucleic acid fusion, for instance two, three, four, five or even more nucleic acid fusions. If two or more nucleic acid fusions are transformed into a host cell, the nucleic acid fusions preferably encode different recombinant polypeptides as described herein. If a host cell transformed with several nucleic acid fusions is used, these nucleic acid fusions may be located on a single vector or on a plurality of vectors.

At least one host cell as described herein, for instance two, three, four, five, six or even more host cells as described herein may be provided in the methods of the present invention. Each of these host cells is transformed with one nucleic acid fusion or at least one nucleic acid fusion, as described herein. Preferably, the nucleic acid fusions transformed in the at least one host cell encode different recombinant polypeptides as described herein.

The different recombinant polypeptides which may be provided in one or at least one host cell may form a functional unit, for instance the subunits of a functional unit, such as the subunits of an enzyme or the subunits or/and components of an enzyme complex.

The nucleic acid fusion comprises (i) a portion encoding a signal peptide, preferably a portion coding for a Gram-negative signal peptide allowing for transport into the periplasm through the inner cell membrane. The signal peptide may be a signal peptide homologous to the host cell. The signal peptide may also be a signal peptide heterologous to the host cell.

Further, the nucleic acid fusion comprises (ii) a portion encoding the recombinant polypeptide to be displayed.

Further, the nucleic acid fusion optionally comprises a portion encoding a protease recognition site, which may be a recognition site for an intrinsic protease, i.e. a protease naturally occurring in the host cell, or an externally added protease. For example, the externally added protease may be an IgA protease (cf. EP-A-0 254 090), thrombin or factor X. The intrinsic protease may be e.g. selected from OmpT, OmpK or protease X.

Furthermore, the nucleic acid fusion comprises (iii) a portion encoding a transmembrane linker which is required for the presentation of the passenger polypeptide (ii) on the outer surface of the outer membrane of the host cell. A transmembrane linker domain may be used which is homologous with regard to the autotransporter, i.e. the transmembrane linker domain is encoded by a nucleic acid portion directly 5' to the autotransporter domain. Also a transmembrane linker domain may be used which is heterologous with regard to the autotransporter. The length of the transmembrane linker is preferably 30-160 amino acids.

Further, the nucleic acid fusion comprises (iv) a transporter domain of an autotransporter. In the context of the present invention, autodisplay may be the recombinant surface display of proteins or polypeptides by means of an autotransporter in any Gram-negative bacterium. The transporter domain of the autotransporter according to the invention can be any transporter domain of an autotransporter and is preferably capable of forming a β-barrel structure. A detailed description of the β-barrel structure and preferred examples of β-barrel autotransporters are disclosed in WO97/35022 incorporated herein by reference. Henderson et al. (2004) describes autotransporter proteins which comprise suitable autotransporter domains (for summary, see Table 1 of Henderson et al., 2004). The disclosure of Henderson et al. (2004) is included herein by reference. For example, the transporter domain of the autotransporter may be selected from Ssp (P09489, S. *marcescens*), Ssp-h1 (BAA33455, S. *marcescens*), Ssp-h2 (BAA11383, S. *marcescens*), PspA (BAA36466, *P. fluorescens*), PspB (BAA36467, *P. fluorescens*), Ssa1 (AAA80490, *P. haemolytica*), SphB1 (CAC44081, *B. pertussis*), AspA/NalP (AAN71715, *N. meningitidis*), VacA (Q48247, *H. pylori*), AIDA-I (Q03155, *E*. *coli*), IcsA (AAA26547, S. *flexneri*), MisL (AAD16954, S. *enterica*), TibA (AAD41751, *E*. *coli*), Ag43 (P39180, *E*. *coli*), ShdA (AAD25110, S. *enterica*), AutA (CAB89117, *N. meningitidis*), Tsh (I54632, *E*. *coli*), SepA (CAC05786, S. flexneri), EspC (AAC44731, *E*. *coli*), EspP (CAA66144, *E*. *coli*), Pet (AAC26634, *E*. *coli*), Pic (AAD23953, *E*. *coli*), SigA (AAF67320, S. flexneri), Sat (AAG30168, *E*. *coli*), Vat (AAO21903, *E*. *coli*), EpeA (AAL18821, *E*. *coli*), EatA (AAO17297, *E*. *coli*), Espl (CAC39286, *E*. *coli*), EaaA (AAF63237, *E*. *coli*), EaaC (AAF63038, *E*. *coli*), Pertactin (P14283, B. *pertussis*), BrkA (AAA51646, *B. pertussis*), Tef (AAQ82668, *B*. *pertussis*), Vag8 (AAC31247, *B. pertussis),* PmpD (084818, C. *trachomatis*), Pmp20 (Q9Z812, C. *pneumoniae*), Pmp21 (Q9Z6U5, C. *pneumoniae),* IgA1 protease (NP_283693, *N. meningitidis),* App (CAC14670, *N. meningitidis),* IgA1 protease (P45386, *H. influenzae*), Hap (P45387, *H. influenzae*), rOmpA (P15921, *R. rickettsii*), rOmpB (Q53047, *R. rickettsii*), ApeE (AAC38796, S. *enterica*), EstA (AAB61674, *P. aeruginosa),* Lip-1 (P40601, X. *luminescens),* McaP (AAP97134, M. *catarrhalis*), BabA (AAC38081, *H. pylori*), SabA (AAD06240, *H. pylori*), AlpA (CAB05386, *H. pylori*), Aae (AAP21063, A. *actinomycetemcomitans*), NanB (AAG35309, *P. haemolytica*), and variants of these autotransporters. Given in brackets for each of the exemplary autotransporter proteins are examples of suitable genbank accession numbers and species from which the autotransporter may be obtained. Preferably the transporter domain of the autotransporter is the *E*. *coli* AIDA-I protein or a variant thereof, such as e.g. described by Niewert U., Frey A., Voss T., Le Bouguen C., Baljer G., Franke S., Schmidt MA. The AIDA Autotransporter System is Associated with F18 and Stx2e in Escherichia coli Isolates from Pigs Diagnosed with Edema Disease and Postweaning Diarrhea. Clin. Diagn. Lab. Immunol. 2001 Jan, 8(1):143-149;9.

Variants of the above indicated autotransporter sequences can e.g. be obtained by altering the amino acid sequence in the loop structures of the β-barrel not participating in the transmembrane portions. Optionally, the nucleic acid portions coding for the surface loops can be deleted completely. Also within the amphipathic β-sheet conserved amino exchanges, i.e. the exchange of an hydrophilic by another hydrophilic amino acid or/and the exchange of a hydrophobic by another hydrophobic amino acid may take place. Preferably, a variant has a sequence identity of at least 70%, at least 90%, at least 95% or at least 98% on the amino acid level to the respective native sequence of the autotransporter domain, in particular in the range of the β-sheets.

Step (b) of the methods of the present invention refers to culturing the host cell under conditions wherein the nucleic acid fusion is expressed and the expression product comprising the recombinant polypeptide is displayed on the surface of the host cell. The person skilled in the art knows suitable culture conditions. The method according to the invention allows for an efficient expression of passenger proteins on the surface of host cells, particularly *E*. *coli* or other Gram-negative bacterial cells up to 100 000 or more molecules per cell by using a liquid medium of the following composition: 5 g/l to 20 g/l, preferably about 10 g/l trypton, 2 g/l to 10 g/l, preferably about 5 g/l yeast extract, 5 g/l to 20 g/l, in particular about 10 g/l NaCl and the remaning part water. The medium should possibly contain as little as possible divalent cations, thus preferably Aqua bidest or highly purified water, e.g. Millipore water is used. The liquid medium may contain in addition preferably EDTA in a concentration of 2 µM to 20 µM, in particular 10 µM. Moreover, it contains preferably reducing reagents, such as 2-mercapto ethanol or dithiotreitol or dithioerythritol in a preferred concentration of 2 mM to 20 mM. The reducing reagents favour a non-folded structure of the polypeptide during transport. The liquid medium can further contain additional C-sources, preferably glucose, e.g. in an amount of up to 10 g/l, in order to favour secretion i.e. transfer of the passenger to the surrounding medium. For surface display preferably no additional C-source is added. Preferred culture conditions for Gram-negative cells, such as *E*. *coli,* are described in the Examples.

If the host cell is a Gram-negative bacterium, the polypeptide synthesized in the cytoplasma can be translocated from the cytoplasm into the periplasmic space by crossing the inner membrane. This can be effected by the signal peptide.

While not wishing to be bound by theory, display of a functional polypeptide comprising a metal porphyrin or a flavin on the surface of a Gram-negative cell by autodisplay can be explained as follows. In a first step the prosthetic group comprising a metal porphyrin or a flavin is introduced into the polypeptide of the present invention in the periplasmic space. In a second step, the recombinant polypeptide of the present invention is translocated from the periplasmic space onto the cell surface in a conformation capable of retaining the prosthetic group when crossing the outer membrane, for example via the omp85 pathway. By this procedure, a functional polypeptide attached to the cell surface can be obtained. In a different mechanism, the prosthetic group, present in the periplasmic space, may be transported independently from the recombinant polypeptide across the outer membrane. A suitable transporter is the outer membrane channel-tunnel protein TolC, in particular for the transportation of metal porphyrins. Both mechanisms may account for transportation of at least a part of prosthetic group transported to the cell surface.

In the present invention, the prosthetic group can be transported to the cell surface by any suitable transport protein, which may be recombinantly expressed in the host cell. This transport can be independent from the autotransporter. The prosthetic group being a metal porphyrin can preferably be transported to the cell surface by a TolC-dependent mechanism. The prosthetic group being a metal porphyrin can also be transported to the cell surface by TolC or/and another suitable transport protein. In Gram negative cells, the prosthetic group being a metal porphyrin can preferably be transported across the outer membrane surface by TolC.

In the present invention, any TolC polypeptide may be employed. For example, an E. coli TolC may be employed.

In particular, the TolC polypeptide is homologous to the host cell. For example, an E. coli TolC may be employed in an E. coli host cell.

The TolC polypeptide may be a recombinant TolC. For example, the TolC polypeptide may be recombinantly expressed in the host cell. TolC may be over-expressed in the host cell. If, for example, the host cell has only low expression of TolC and thus only low capability of porphyrin transport to the cell surface, TolC may be over-expressed.

The TolC polypeptide, as used herein, may comprise a sequence selected from
(a) SEQ ID NO:8, and
(b) sequences having at least 70%, at least 80%, at least 90%, at least 95%, or at least 98% identity to the sequence of (a).

The TolC polypeptide, as used herein, may be encoded by a sequence selected from
(a) nucleic acid sequences encoding the amino acid sequences of SEQ ID NO:8,
(b) nucleic acid sequences encoding amino acid sequences having at least 70%, at least 80%, at least 90%, at least 95%, or at least 98% identity to the amino acid sequence of SEQ ID NO:8,
(c) SEQ ID NO:7, and
(d) sequences having at least 70%, at least 80%, at least 90%, at least 95%, or at least 98% identity to the sequence of (c).

In particular, nucleic acid sequences of (a), (b) and (d) include sequences within the scope of the degeneracy of the genetic code.

The TolC polypeptide, as defined herein, may be a HasF polypeptide, for example from Serratia marcescens.

If the passenger polypeptide is transported together with the prosthetic group to the cell surface, the passenger may acquire the prosthetic group within the cell. In the method of the present invention, in step (b), the prosthetic group endogenously produced in the cell may be introduced into the polypeptide of the present invention within the periplasmic space.

In the method of the present invention, step (b) may involve the omp85 pathway. Step (b) may comprise transportation of the polypeptide of the present invention via the omp85 pathway. Step (b) may comprise translocation of the polypeptide of the present invention from the periplasmic space onto the cell surface by the omp85 pathway, in particular in a conformation capable of retaining the prosthetic group when crossing the outer membrane.

In the present invention, any Omp85 or Omp85 homologue may be employed. "Omp85", as used herein, includes homologues of Omp85. For example, the Omp85 homologue YaeT from *E*. *coli* may be employed.
The Omp85, in particular YaeT, may comprise a sequence selected from
(a) SEQ ID NO:3 and SEQ ID NO:4, and
(b) sequences having at least 70%, at least 80%, at least 90%, at least 95%, or at least 98% identity to the sequence of (a).

Also employed may be a nucleic acid encoding an Omp85. The nucleic acid encoding Omp85, in particular YaeT, may comprise a nucleic acid sequence selected from
(a) nucleic acid sequences encoding the amino acid sequence of SEQ ID NO:3 and SEQ ID NO:4, and
(b) nucleic acid sequences encoding amino acid sequences having at least 70%, at least 80%, at least 90%, at least 95%, or at least 98% identity to the amino acid sequence of SEQ ID NO:3 or/and SEQ ID NO:4,

In particular, nucleic acid sequences of (a) and (b) include sequences within the scope of the degeneracy of the genetic code.

The components (i) (ii), the optional portion encoding a protease recognition site, (iii) and (iv) in the nucleic acid fusion of the present invention are preferably oriented from 5' to 3'. In the expression product obtained in step (b), the amino acid sequences encoded by nucleic acid sequences (i) and (ii), an optional protease recognition site as described herein, the amino acid sequences encoded by nucleic acid sequences (iii) and (iv) are preferably arranged N terminal to C terminal.

The method of the present invention may comprise preparing a membrane preparation from the cell obtained in step (b). The membrane preparation may comprise membrane particles. The membrane particles may be membrane vesicles. Preferred membrane particles are outer membrane particles. In particular the method of the present invention may comprise preparing outer membrane particles of cells displaying a recombinant polypeptide on the surface, e.g. of Gram-negative bacterial cells. The person skilled in the art knows suitable conditions (e.g. Hantke, 1981, Schultheiss et al., 2002). Typical conditions for preparing membrane particles are employed in the examples of the present invention. Outer membrane particles from a host cell as described herein may be performed by a method comprising the steps:
(a) treating the host cell with a hydrolase (such as lysozyme) and optionally with a DNAse. This enzymatic treatment may be performed at room temperature. The hydrolase hydrolyses the cell wall within the periplasmic space. The cell wall comprises peptidoglycans to be hydrolyzed.
(b) optionally solubilizing the preparation of (a) with a tenside, such as Triton X-100, or/and with sarcosine, followed by optional centrifugation of cell debris. The thus obtained preparation of outer membrane particles may be centrifuged, washed and resuspended.

The diameter of the membrane particles may be in the range of 1 nm to 1000 nm, in the range of 50 nm to 500 nm, in the range of 75 to 200 nm, or in the range of 90 to 120 nm. At least 80%, at least 90%, at least 95 %, or at least 98% of the membrane particles may have a diameter in a range selected from the ranges described herein.

In a host cell being a Gram-negative bacterium, such as *E*. *coli,* after translocation, the recombinant passenger remains attached to the surface of the outer membrane by the β-barrel, which is serving as an anchor within the outer membrane. Due to the controlled integration of the β-barrel within the outer membrane, the C terminal part of the β-barrel is directed to the inner side of the outer membrane, whereas the N-terminal part of the linker, to which the recombinant passenger protein is covalently bound, is directed to the outer surface of the outer membrane, i.e. the environment. The recombinant passenger protein has an oriented location after transport, namely it is directed to the cellular surface. The recombinant passenger protein has the identical orientation as the lipopolysaccharide (LPS) layer which may be present in the outer membrane.

Membrane particles of the present invention prepared from the host cell of the present invention comprise the recombinant peptide at the surface directed to the environment. In contrast to the inner membrane which is a unit membrane, the outer membrane of Gram-negative bacteria, in particular *E*. *coli,* is asymmetric. The outer membrane may comprise an inner layer comprising phospholipids and an outer layer comprising LPS. LPS is hydrophilic and may contain several negative charges. By using outer membrane particles with anchored passenger proteins by a β-barrel for the coating of carriers, the outer side of the outer membrane, in particular the LPS side will be directed to the surface distal to the carrier. As a consequence the recombinant protein or a domain thereof, which are integrated in the outer membrane by autodisplay, will be directed to the surface distal to the carrier as well. The core part of the membrane particles may stabilize the interaction of the outer membrane layer obtained by applying outer membrane particles to the carrier by hydrophobic interactions and may contain lipoproteins or peptidoglycans.

A preferred prosthetic group is a metal porphyrin, as described herein.

The prosthetic group being the metal porphyrin may comprise a heavy metal such as cobalt, nickel, manganese, copper and iron. The metal porphyrin of the present invention in particular comprises a heme group.

Another preferred prosthetic group is a flavin, as described herein.

The prosthetic group being the flavin may be selected from FAD and FMN.

The polypeptide of the present invention comprising a prosthetic group preferable is an enzyme.

The polypeptide comprising the metal porphyrin may be an enzyme. The polypeptide comprising the metal porphyrin may be selected from P450 enzymes (such as P450 reductases) and cytochromes (such as cytochrome b5). The polypeptide comprising the metal porphyrin may be selected from hemoproteins. In particular, the polypeptide comprising the metal porphyrin may be selected from monooxygenases. The polypeptide comprising the metal porphyrin may be selected from CYP11B1, CYP11A1, CYP106A2 and CYP3A4. The polypeptide comprising the metal porphyrin may preferably be selected from CYP106A2 and CYP3A4.

In a preferred embodiment, the polypeptide comprising the metal porphyrin comprises a sequence selected from
(a) SEQ ID NO:2 and SEQ ID NO:6, and
(b) sequences having at least 70%, at least 80%, at least 90%, at least 95%, or at least 98% identity to the sequence of (a).

The portion (ii) of the nucleic acid fusion of the present invention may encode a polypeptide which, when functional, comprises the metal porphyrin, as described herein. The portion (ii) of the nucleic acid fusion of the present invention may comprise a nucleic acid sequence selected from
(a) nucleic acid sequences encoding the amino acid sequences of SEQ ID NO:2 and SEQ ID NO:6,
(b) nucleic acid sequences encoding amino acid sequences having at least 70%, at least 80%, at least 90%, at least 95%, or at least 98% identity to the amino acid sequence of SEQ ID NO:2 or/and SEQ ID NO:6,
(c) SEQ ID NO:1 and SEQ ID NO:5, and
(d) sequences having at least 70%, at least 80%, at least 90%, at least 95%, or at least 98% identity to the sequence of (c).
In particular, nucleic acid sequences of (a), (b) and (d) include sequences within the scope of the degeneracy of the genetic code.

The skilled person knows suitable methods to determine the degree of identity of nucleic acid sequences and amino acid sequences. Known algorithms, such as BLAST (for nucleic acids) or PBLAST (for amino acid sequences) may be used. A nucleic acid or polypeptide comprising sequences having at least 70%, at least 80%, at least 90%, at least 95%, or at least 98% identity to a given sequence includes fragments of the given nucleic acid or polypeptide.

The flavin containing polypeptide of the present invention may be selected from flavoproteins, in particular FAD or FMN containing proteins. Preferred are FAD containing proteins. The flavin containing polypeptide may be selected from enzymes such as oxidoreductases, NADH oxidases, dehydrogenases, and oxidases, especially sugar oxidases, such as pyranose oxidase. The NADH oxidase is in particular an FAD containing enzyme.

The polypeptide of the present invention to be displayed on the surface of the cell may be a multimeric polypeptide. The multimeric recombinant polypeptide may be a homodimer, i.e. a polypeptide consisting of two identical subunits or a homomultimer, i.e. a polypeptide consisting of three or more identical subunits. On the other hand, the multimeric recombinant polypeptide may be a heterodimer, i.e. a polypeptide consisting of two different subunits or a heteromultimer consisting of three or more subunits wherein at least two of these subunits are different. For example, the multimeric polypeptide is comprised of a plurality of subunits which form a "single" multimeric polypeptide or a complex of a plurality of functionally associated polypeptides which may in turn be monomeric and/or multimeric polypeptides. It should be noted that at least one subunit of the multimeric recombinant protein may contain at least one prosthetic group as described herein. Further, is should be noted that the nucleic acid fusion may encode a plurality of polypeptide subunits as a polypeptide fusion which when presented on the cell surface forms a functional multimeric polypeptide.

Homodimers or homomultimers may be formed by a spontaneous association of several identical polypeptide subunits displayed on the host cell membrane. Heterodimers or heteromultimers may be formed by a spontaneous association of several different polypeptide subunits displayed on the host cell membrane.

On the other hand, a multimeric recombinant polypeptide may be formed by an association of at least one polypeptide subunit displayed on the host cell membrane, as described herein, and at least one soluble polypeptide subunit added to the host cell membrane. The added subunit may be identical to the displayed subunit or be different therefrom.

Yet another aspect of the present invention is a host cell displaying the recombinant polypeptide on the surface. The host cell may be any host cell as described herein, in particular a host cell displaying a recombinant polypeptide on the surface thereof, wherein the recombinant polypeptide contains a prosthetic group comprising a metal porphyrin or a flavin, and wherein the recombinant polypeptide comprises
(I) a portion comprising the recombinant polypeptide to be displayed,
(II) a portion comprising a transmembrane linker, and
(III) a portion comprising the transporter domain of an autotransporter,
wherein the host cell recombinantly expresses a transport protein capable of transporting the prosthetic group to the host cell surface, and wherein the prosthetic group is transported to the host cell surface by the transport protein independently from the recombinant polypeptide. Optionally, the recombinant polypeptide comprises a portion comprising a protease recognition site.

The displayed polypeptide is in particular a functional polypeptide.

The portions (I) to (III) of the recombinant polypeptide displayed by the host cell of the present invention are encoded in particular by the components (ii), (iii), and (iv) of the nucleic fusion, as described herein. The optional portion comprising a protease recognition site is encoded in particular by the optional portion encoding a protease recognition site of the nucleic acid fusion described herein.

Yet another aspect of the present invention is a membrane preparation comprising a recombinant polypeptide. The membrane preparation of the present invention may comprise membrane particles, as described herein.The membrane preparation may be obtained from a host cell as described herein. The recombinant polypeptide of the may be any recombinant polypeptide as described herein.

Yet another aspect of the present invention is the use of a membrane preparation comprising a recombinant polypeptide in the manufacture of a carrier comprising a recombinant polypeptide.

The membrane preparation of the present invention may be employed for coating a carrier. The carrier may comprise a membrane preparation of the present invention, as described herein.

The carrier may comprise a hydrophobic surface. The hydrophobic surface may have a contact angle of more than 90°. A increasing surface angle of more than 30° indicates a gradually increasing hydrophobicity of a surface. In the present context, a hydrophobic surface may have a contact angle of at least 40°. The surface preferably has a hydrophobicity described by a contact angle of at least 40°, at least 50°, at least 60°, at least 65°, at least 70°. Contact angles are preferably determined by the sessile drop method. The sessile drop method is a standard method for determining contact angles. Measurements may be performed with a contact angle goniometer. Preferred contact angles of the hydrophobic surface are in a range of 40° to 100°, 50° to 90°, or 60° to 80°.
The surface of the carrier may be a metal surface. A suitable metal surface has a contact angle e.g. in the range of 50° to 80°. A suitable metal may be selected from gold, silver, titanium, aluminium and alloys such as brass. A preferred surface is a gold surface. The gold surface may be employed as it is. An untreated gold surface has a hydrophobicity suitable for the carrier as described herein. A treatment of the gold surface with thiolated hydrocarbons or hydrocarbons with functional groups such as carboxylic acids or hydroxyl groups is not required.

Another preferred surface of the carrier comprises a polymer, for instance a surface usually employed in disposable materials for use in biochemical or/and medical science. The polymer may be an artificial polymer. Examples of artificial polymers include a polymer selected from polystyrenes, polypropylenes, and polycarbonates. The polystyrene may be produced from [2,2]paracyclophane monomers. Polystyrene surfaces may be treated with oxygene plasma introducing OH or/and methylene groups in order to modify the hydrophobicity. Examples of such modified surfaces include Maxi-sorp, Medi-sorp, Multi-sorp, and Poly-sorp surfaces. Another suitable polystyrene surface is Parylene N produced from [2,2]paracyclophane monomers. Yet another suitable surface is Parylene A [Poly(monoamino-p-xylene)]. Especially suitable are surfaces comprising a polymer having a hydrophobicity described by a contact angle of at least 50°. Suitable surfaces are selected from polystyrene, Parylene A, Parylene N, Maxi-sorp, Medi-sorp, Multi-sorp, and Poly-sorp. Preferred surfaces are selected from polystyrene, Parylene A, Parylene N, Maxi-sorp, Medi-sorp, and Poly-sorp.

The surface may comprise a natural polymer. Suitable natural polymers include polybutyrate and cellulose and derivatives thereof. A further surface is provided by latex particles, in particular latex beads.

Yet another surface is provided by C18-modified particles, in particular C18-modified monolithic silica particles. C18 refers to an alkyl group comprising 18 carbon atoms. C18-modified particles are known in the art.

Yet another suitable surface is a glass surface.

The surface may be modified is order to adjust the hydrophobicity. Modification may be performed by chemical treatment (i.e. by oxygen plasma), physical treatment (e.g. by laser irradiation or/and radioactive irradiation), or by mechanical treatment.

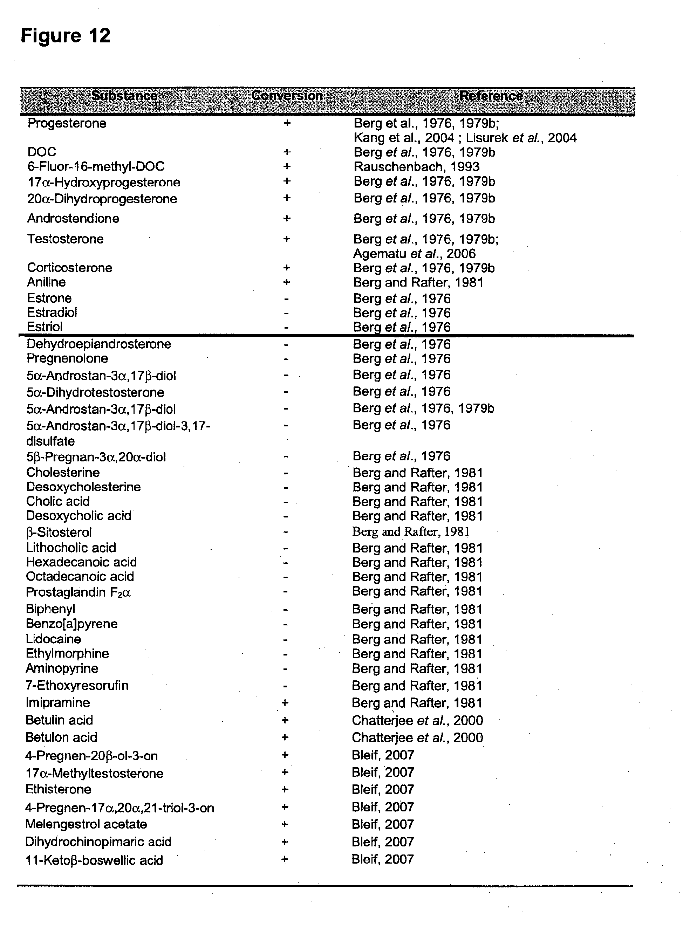

The method according to the invention and the host cells according to the invention can be used for a variety of different applications, e.g. as whole cell biofactories or membrane preparation biofactories for chemical synthesis procedures, e.g. for the synthesis of organic substances selected from enzyme substrates, drugs, hormones, starting materials and intermediates for syntheses procedures and chiral substances (cf. Roberts, Chemistry and Biology 6 (1999), R269-R272). Typical CYP106A2 substrates are described in Fig. 12.

In particular, the method according to the invention and the host cells according to the invention, as described herein, can be used in the chemical synthesis, for example in enzymatically catalyzed enantioselective or/and regioselective steps. For example, CYP106A2 displayed on the surface of a cell, as described herein, can be used for the conversion of steroids, for the conversion of abietic acid, or for the preparation of desipramine from imipramine, as exemplified by Examples 1 and 3.

Furthermore, the cell or the membrane preparation of the invention may be used for a directed evolution procedure, e.g. for the development of new biocatalysts for the application in organic syntheses.

This is achieved in a particular embodiment by varying the amino acid sequence of the polypeptide containing a prosthetic group selected from metal porphyrins and flavins, as described herein, via site-specific or random mutagenesis and by testing variant carrying cells or membrane preparations or libraries containing variant carrying cells or membrane preparations thereof using a certain chemical reaction with the help of suitable screening methods, in particular high throughput screening (HTS) methods for the conversion of a certain substrate.

In yet another preferred embodiments libraries of variants of a polypeptide containing a prosthetic group selected from metal porphyrins and flavins, as described herein, are examined in view of the role of defined amino acids during certain functions, in particular catalytic functions.

In general, these particular embodiments concern the production of variants of proteins and/or enzymes and the production of libraries with variants of proteins and/or enyzmes, respectively, which carry a prosthetic group, as described herein, or multimers etc. and which are screened in view of a certain characteristic, i.e. one or optionally several variants fulfilling this desired characteristic perfectly are selected. By selecting the variant the cell is selected, too, and carries the nucleic acid coding the variant. Thus, at the same time both the amino acid sequence and the structural information of the variant can be determined via the nucleic acid sequence. The characteristics in question are particularly enzyme inhibiting, catalytical, toxin degrading, synthesizing, therapeutical etc. characteristics.

Moreover, the host cell or the membrane preparation may be used as an assay system for a screening procedure, e.g. for identifying modulators (activators or inhibitors) of displayed polypeptides, containing a prosthetic group selected from metal porphyrins and flavins, as described herein, which may be used as potential therapeutic agents. The screening procedure may also be used to identify variants of displayed polypeptides having predetermined desired characteristics. For this purpose, libraries of modulators and/or libraries of displayed polypeptides may be used. Further, the host cells or membrane preparations derived therefrom may be used as a system for toxicity monitoring and/or degrading toxic substances in the environment, in the laboratory or in biological, e.g. human, animal, or non-biological systems.

An essential advantage of applying the host cells and membranes according to the invention is enabling correct folding and biological activity of proteins or protein complexes, e.g. of the polypeptide containing a prosthetic group selected from metal porphyrins and flavins, as described herein, which require a membrane environment. Thus, a reconstitution as previously considered to be necessary is no longer required. Thereby the production steps of a functional biocatalytic system are simplified and an increased stability of the system per se is obtained.

Further preferred examples for the recombinant polypeptide to be displayed, i.e. the passenger polypeptides are peptides or proteins selected from the group of drug metabolizing enzymes, such as CYP1A2 involved in the activation of aromatic amine carcinogenes, heterocyclic arylamine promutagenes derived from food pyrolysates and aflatoxin B1 (Gallagher EP, Wienkers LC, Stapleton PL, Kunze KL, Eaton DL., Role of human microsomal and human complementary DNA-expressed cytochromes P4501A2 and P4503A4 in the bioactivation of aflatoxin B1. Cancer Res. 1994, Jan 1;54(1):101-8) or CYP2E1 capable of activating the procarcinogenes N-nitrosodimethylamine and N-nitrosodiethylamin and metabolizes the procarcinogenes benzene, styrene, carbon tetrachloride, chloroform (Yoo JS, Ishazaki H, Yang CS., Roles of cytochrome P450IIE1 in the dealkylation and denitrosation of N-nitrosodimethylamine and N-nitrosodiethylamine in rat liver microsomes. Carcinogenesis. 1990 Dec; 11(12):2239-43; Peter R, Bocker R, Beaune PH, Iwaskai M, Guengerich FP, Yang CS., Hydroxylation of chlorzoxazone as a specific probe for human liver cytochrome P-450IIE1. Chem. Res. Toxicol. 1990 Nov-Dec;3(6):566-73). Further preferred passenger peptides are peptides from the group of steroid biosynthesis enyzmes, such as CYP11B1 involved in the formation of cortisol and aldosterone (Bernhardt R., Cytochrome P450: structure, function and generation of reactive oxygen species. Rev. Physiol. Biochem. Pharmacol. 1996;127:137-221) or CYP19 involved in the conversion of adrostenedione to 19-hydroxyandrostenedione, 19-oxo-androstenedione and estrone (Ryan KJ., Biological aromatization of steroids. J. Biol. Chem. 1959;134:268). Further preferred metal ion containing enzymes are Cu-containing enzymes, such as cytochrome-oxidase, Mn-containing enzymes, such as arginase and ribonucleotide reductase, Mo-containing enzymes, such as dinitrogenase and Se-containing enzymes, such as glutathione peroxidase.

Preferably the P450 enzymes are hepatic P450 enzymes, particularly P450 3A4, 2D6, 2C9 and 2C19. The host cells and/or preparations according to the invention are preferably used sequentially for testing the enzyme inhibition of P450 enzymes. For example, with the help of the host cell and/or membrane preparation according to the invention it can be found out in an early step of drug discovery, the so-called lead identification, whether the new drug lead structure to be tested could possibly have side-effects or lead to the so-called drug-drug interaction.

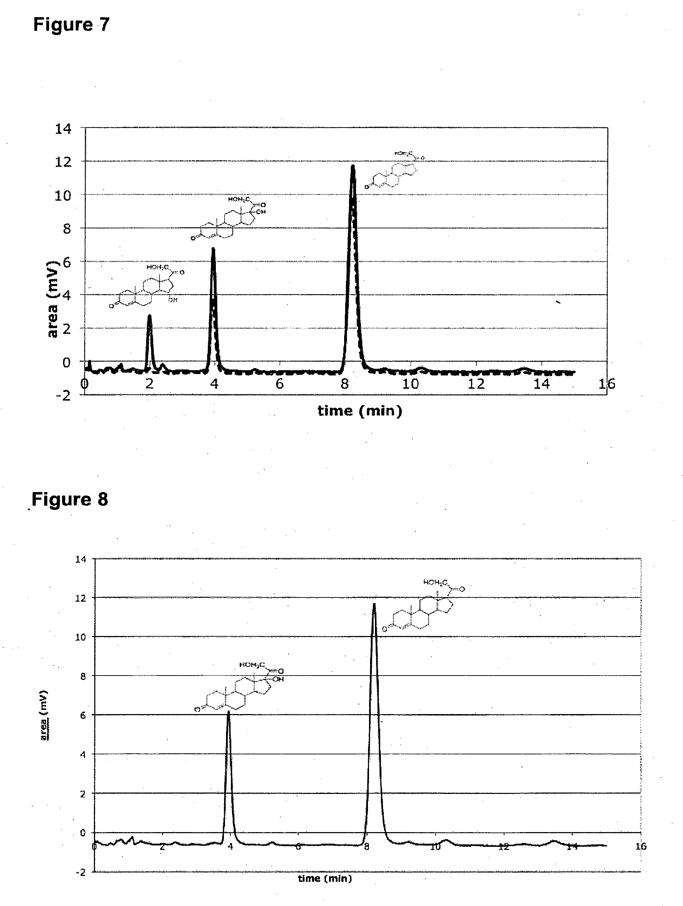

Further, the present invention shall be further illustrated by the following figures and examples:
- **Figure 1:**: **A:** Schematic drawing of the hydroxylation reaction in the 15β-position of the steroid 11-deoxycorticosterone (DOC) catalyzed by CYP106A2. Redox equivalents are transferred from NADPH via the proteins AdR and Adx to the steroid converting enzyme CYP106A2. **B**: Oxidation of terfenadine by CYP3A4 to fexofenadine.
- **Figure 2:**: Chromatogram of the CYP106A2 activity assay. Conversion of 11-deoxycorticosterone into 15beta-deoxycorticosterone using the pure enzyme. (Reichstein's Compound S, RSS, internal standard)

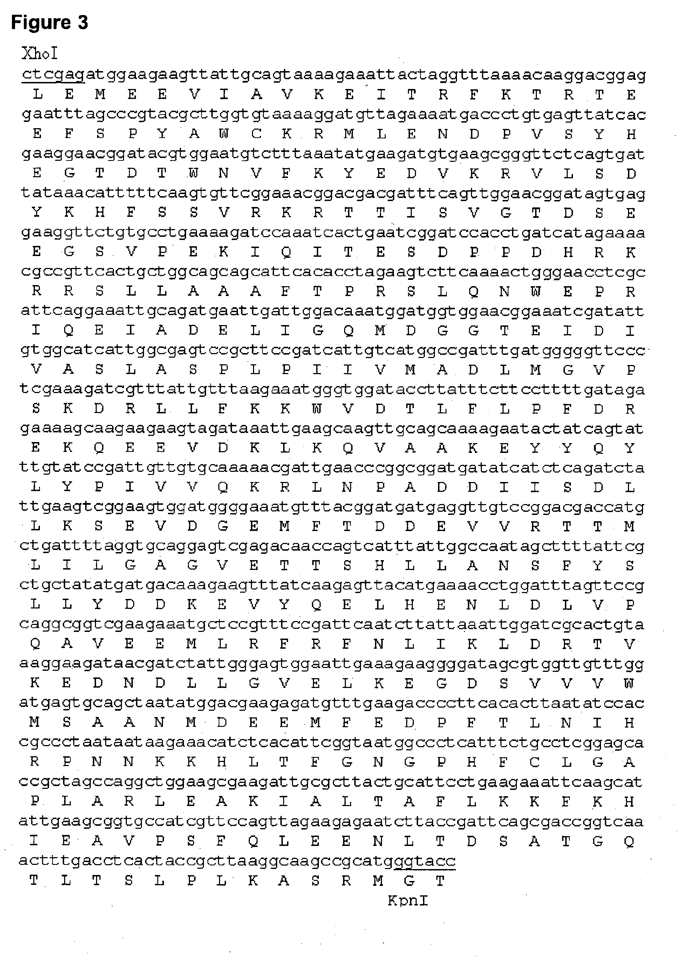

- **Figure 3:**: Sequence of CYP106A2. Nucleic acid sequence (SEQ ID NO:1) and derived amino acid sequence (SEQ ID NO:2) of the CYP106A2 insert in plasmid pET-CYP13.
- **Figure 4:**: **A:** Secretion mechanism of the autotransporter proteins in Gram-negative bacteria. **B**: Structure of a typical artificial autotransporter protein used in autodisplay (SEQ ID NO:9 and SEQ ID NO:10). **C:** Structure of the CYP106A2 fusion protein. Illustration of the fusion proteins necessary for the expression of CYP106A2. Important restriction sites for cloning are underlined (SEQ IDs NO:11-14).
- **Figure 5:**: Expression of CYP106A2. SDS-PAGE (10 %) and Coomassie staining of outer membrane preparations obtained form *E*. *coli* BL21(DE3) pET-CYP13. 1: marker proteins, 2: control, BL21(DE3) without plasmid, 3: BL21 (DE3) pET-CYP13-IPTG, 4: BL21 (DE3) pET-CYP13 + 1 mM IPTG.

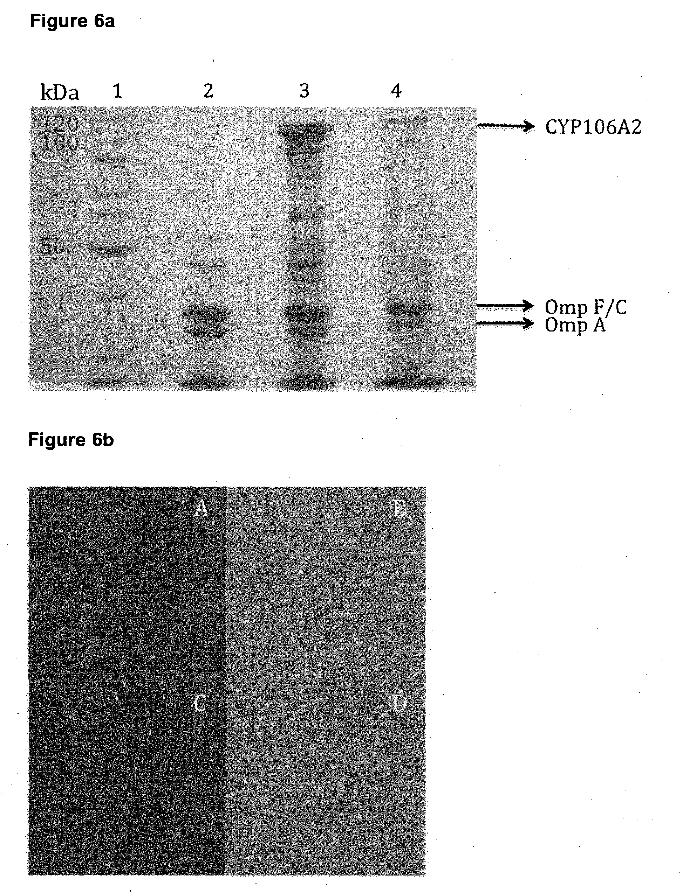

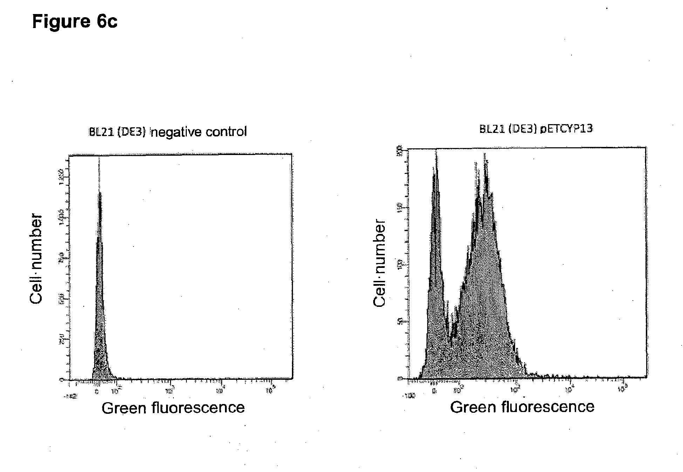

- **Figure 6:**: **a:** Surface display of CYP106A2. Whole cell trypsin digestion and subsequent SDS-PAGE (10 %) and Coomassie staining of outer membrane preparations obtained from *E. coli* BL21 (DE3) pET-CYP13. 1: marker proteins, 2: control, BL21 (DE3) without plasmid, 3: BL21 (DE3) pET-CYP13 + IPTG - trypsin, 4: BL21 (DE3) pET-CYP13 + 1 IPTG + trypsin. **b**: Proof of successful surface display of CYP106A2 by indirect immune fluorescence A: *E*. *coli* BL21 (DE3) pETCYP13, abs.: 490nm, em.: 520nm, B: *E*. *coli* BL21 (DE3) pETCYP13, transmitted light, C: *E*. *coli* BL21 (DE3), abs.: 490nm, em.: 520nm, D: *E*. *coli* BL21 (DE3), transmitted light. All samples were prepared with two antibodies: a primary polyclonal anti- CYP106A2 antibody and a secondary FITC- labelled detection antibody. Only the cells containing the expression plasmid showed a positive reaction (see A). **c**: Proof of successful surface display of CYP106A2 by flow cytometry. All samples were prepared with two antibodies: a primary polyclonal anti- CYP106A2 antibody and a secondary FITC- labelled detection antibody. The mean fluorescence (mF) of the labelled cells was determined by FACS analysis; BL21 (DE3) (negative control), mF = 27; cells displaying CYP106A2 on the surface (BL21(DE3) pETCYP13), mF = 268.
- **Figure 7:**: Chromatogram of the activity assay with whole cells displaying CYP106A2. Conversion of 11-deoxycorticosterone into 15betadeoxycorticosterone by BL21(DE3) pETCYP13. For that purpose cells were cultivated and half of them induced with 1 mM IPTG. Formation of the product only occurs after induced protein expression. (Reichstein's Compound S, RSS, internal standard)
- - - - BL21 (DE3) pET CYP 13 without addition of IPTG;
- BL21(DE3) pET CYP 13 with addition of 1 mM IPTG
- **Figure 8:**: Chromatograms of the CYP106A2 activity assay using BL21(DE3) pETCYP13 cells without addition of Adx (additional negative control). Cells were cultivated and protein expression was induced with 1 mM IPTG. To proof that all conversions took only place on the surface of *E*. *coli* and not inside the cell by other electron supplying systems, substrate conversions were done without the addition of Adx, since it is too large of a molecule to enter the cell envelope. The chromatograms of this conversion assay shows, as expected, no product peak. (Reichstein's Compound S, RSS, internal standard).
- BL21(DE3) pET CYP 13 with addition of 1mM IPTG
- **Figure 9:**: Schematic drawing of the hydroxylation of abietic acid catalysed by CYP106A2. Redox equivalents are transferred from NADPH via the proteins AdR and Adx to CYP106A2.
- **Figure 10:**: Chromatogram of the CYP106A2 activity assay using purified enzyme. Conversion of the non membrane transferrable educt abietic acid into the two products 12-alpha und 12-beta-Hydroxy-abietic acid by the purified enzyme CYP106A2. (Reichstein's Compound S, RSS, internal standard).
- **Figure 11:**: Chromatogram of the activity assay using BL21(DE3) cells displaying CYP106A2. Conversion of the non membrane transferrable educt abietic acid into the two products 12-alpha and 12-beta-hydroxy-abietic acid by BL21(DE3) pETCYP13. For that purpose cells were cultivated and half of them induced with 1 mM IPTG. Formation of the product occurs in particular after induced protein expression. (Reichstein's Compound S, RSS, internal standard).
- - - - BL21(DE3) pET CYP 13 without addition of IPTG;
- BL21 (DE3) pET CYP 13 with addition of 1 mM IPTG
- **Figure 12:**: Table of known CYP106A2 substrates from *Bacillus megaterium* ATCC 13368.

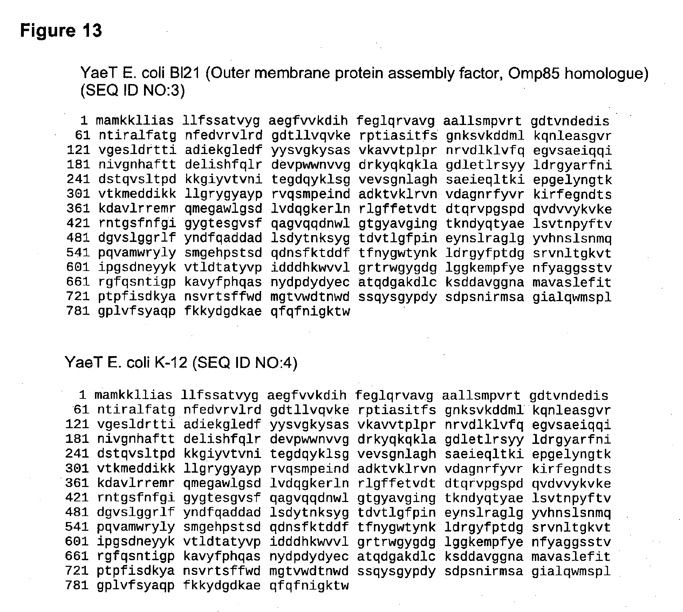

- **Figure 13:**: Sequence of YaeT (Outer membrane protein assembly factor, Omp85 homologue) in E. coli strains BI21 (SEQ ID NO:3) and K-12 (SEQ ID NO:4).

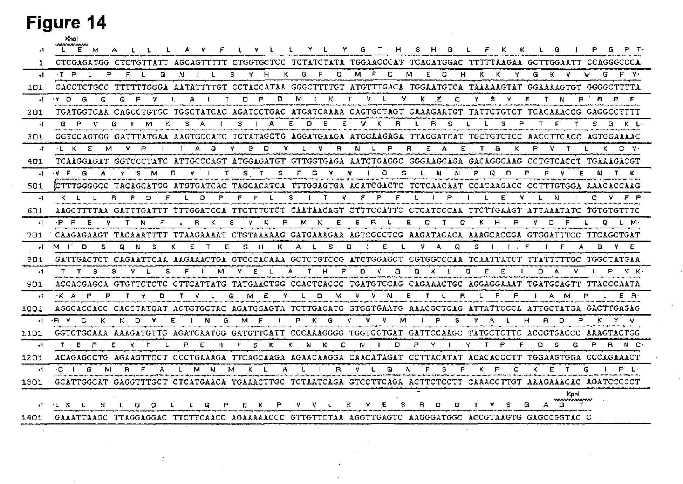

- **Figure 14:**: Sequence of CYP3A4. Nucleic acid sequence (SEQ ID NO:5) and derived amino acid sequence (SEQ ID NO:6) of the CYP3A4 insert in plasmid pSC001 used for autodisplay of CYP3A4.

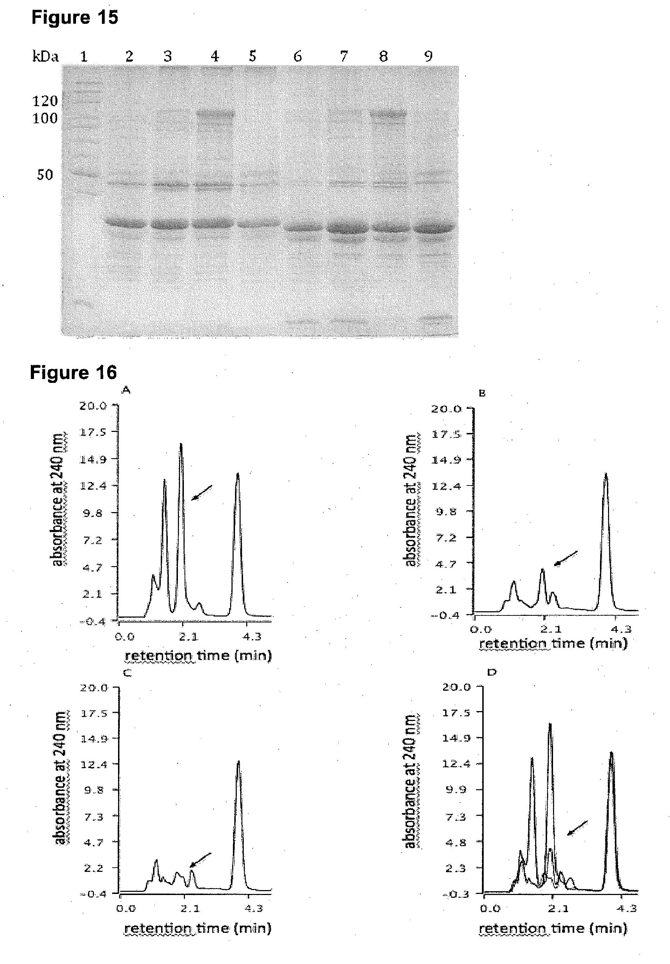

- **Figure 15:**: Surface display of CYP106A2 in TolC negative cells. Whole cell proteinase k digestion and subsequent SDS-PAGE (12.5 %) and Coomassie staining of outer membrane preparations obtained from *E*. *coli* BL21(DE3) pET-CYP13 and JW 5503 (DE3) pETCYP13. **1:** marker proteins, **2:** control, BL21(DE3) without plasmid, **3:** BL21 (DE3) pET-CYP13 - IPTG - proteinase k.**4:** BL21 (DE3) pET-CYP13 + IPTG - proteinase k, **5:** BL21 (DE3) pET-CYP13 + 1 IPTG + proteinase k, **6:** control, JW 5503 (DE3) without plasmid, **7:** JW 5503 (DE3) pET-CYP13 - IPTG-proteinase k **8:** JW 5503 (DE3) pET-CYP13 + IPTG - proteinase k, **9:** JW 5503 (DE3) pET-CYP13 + 1 IPTG + proteinase k.
- **Figure 16:**: HPLC chromatograms showing CYP106A2 conversion of 11-deoxycorticosterone to 15β-deoxycorticosterone **A:** positive control (purified CYP106A2 enzyme). **B**: TolC positive cells (BL21 (DE3) pETCYP13) induced with IPTG. **C:** TolC negative cells (JW 5503 (DE3) pETCYP13) induced with IPTG. **D:** Overlay of the 3 graphs. The major product, 15p-deoxycorticosterone, was seen at a retention time of 2 min. The amount of this product decreased when CYP106A2 was expressed in the *E*. *coli* strain lacking the TolC channel protein. The peak at the retention time of 4 min is the internal standard Reichstein's compound S.
- **Figure 17:**: Schematic drawing of the N-demethylation of the antidepressant imipramine into desipramin. The reaction is catalyzed by CYP106A2 displayed on the surface of *E*. *coli* cells. Redox equivalents are transferred from NADPH via the proteins AdR and Adx to the converting enzyme.
- **Figure 18:**: NADPH consumption of cells by *E*. *coli* BL21(DE3) pETCYP13. Data points are the average of triplicate experiments. The bars represent the standard deviation. Squared symbols represent BL21 (DE3) cells and round symbols represent BL21 (DE3) pETCYP13 cells induced with 1 mM IPTG.

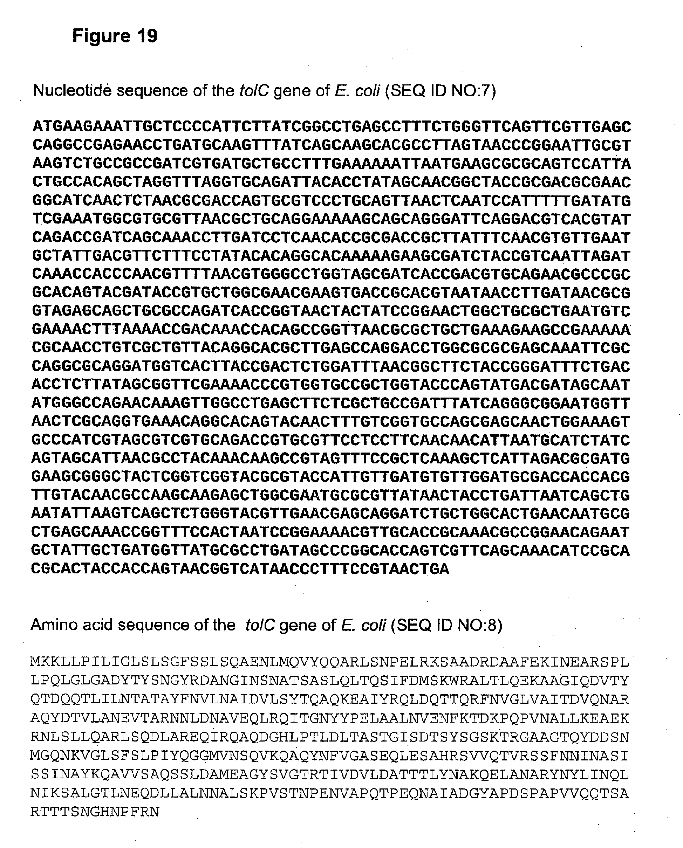

- **Figure 19:**: Sequence of E. coli TolC. SEQ ID NO:7 describes a nucleotide sequence encoding TolC. SEQ ID NO:8 describes a TolC amino acid sequence.

### Example 1

In this example, mainly two P450 enzymes shall be expressed by autodisplay, CYP106A2 and CYP3A4.

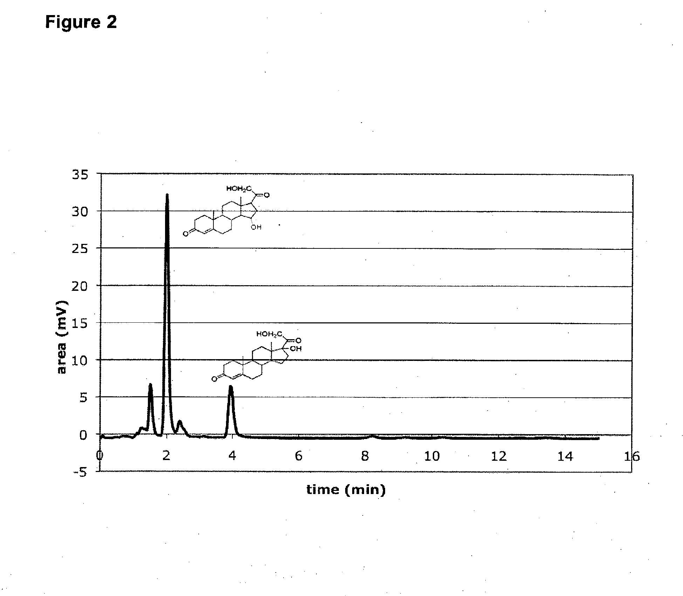

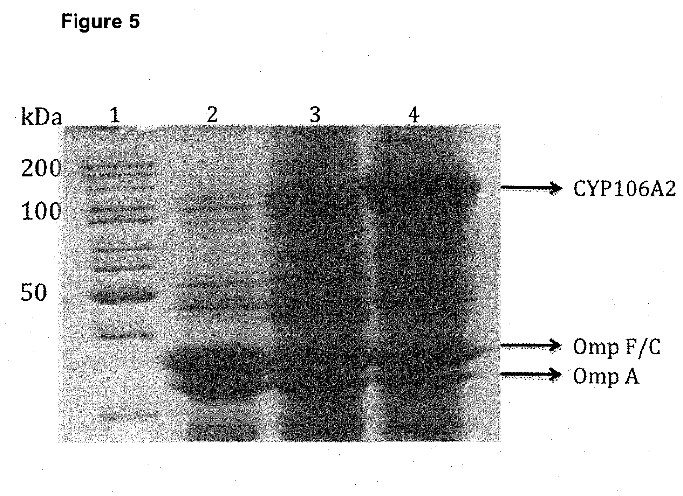

To establish an efficient HPLC analytic method experiments using the purified enzyme were conducted and retention times of the educt, product and internal standard determined. (Fig.1a and Fig. 2) The gene of CYP106A2 was amplified by PCR and inserted into a plasmid encoding the domains needed for autodisplay. Successful expression was shown in an SDS- PAGE (Fig. 5) To find out whether the CYP106A2 domain of the fusion protein was indeed exposed at the cell surface, trypsin was added to whole cells of E. coli BI21(DE3) pET-CYP13 after incubation with 1 mM IPTG for one hour. Trypsin is too large of a molecule to enter the cell envelope of *E*. *coli.* This means, if the CYP106A2 is degraded by trypsin, when added to whole cells, it must be accessible at the cell surface. Because OmpA, which has a N terminal extension in the periplasm, was not digested at all, it could be excluded, that trypsin had entered the periplasm due to cell leakyness. (Fig. 6a) Two additional methods to proof successful surface display came into operation as well; fluorescence microscopy (Fig. 6b) and FACS (Fig. 6c).

To measure if CYP106A4 was indeed expressed on the surface of the cells in a functional form activity tests were conducted. For that purpose cells were cultivated and protein expression induced using 1 mM Isopropyl-beta-D-thiogalactopyranosid (IPTG). In a conversion assay it was tested whether the cells displaying CYP106A2 on the surface have the ability to efficiently convert 11-deoxycorticosterone (DOC) into 15beta-DOC. To enhance the activity of the displayed enzyme adrenodoxin (Adx) and adrenodoxin reductase (AdR) from bovine adrenals, supplying this enzyme with the reducing equivalents necessary for steroid hydroxylation activity, were added. The use of whole *E.coli* cells only resulted in a product peak if protein expression was induced with 1 mM IPTG (Fig. 7). To proof that all conversions took only place on the surface of *E*. *coli* and not inside the cell by other electron supplying systems, substrate conversions were done without the addition of Adx, since it is too large of a molecule to enter the cell envelope. The chromatogram of this conversion assay shows, as expected, no product peak (Fig. 8).

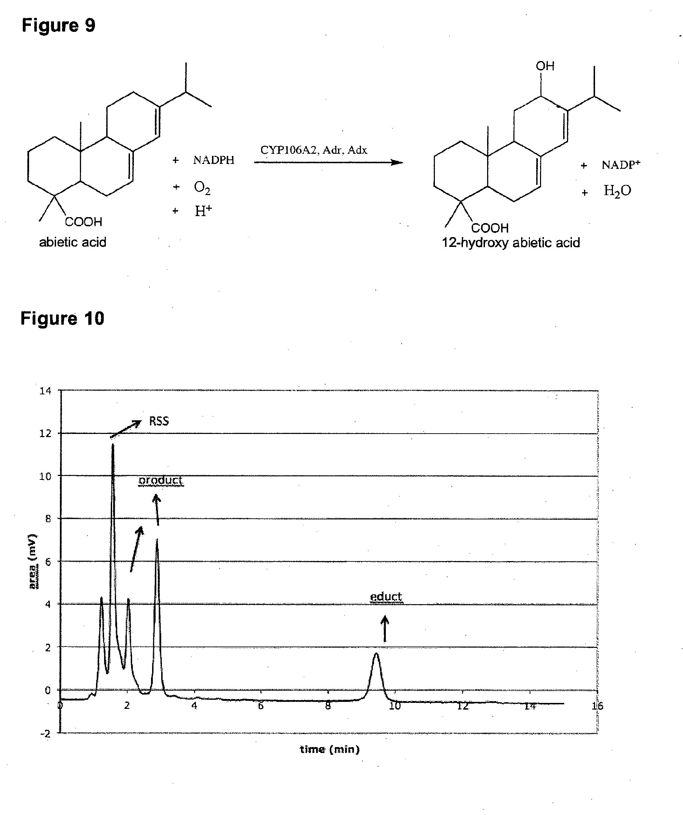

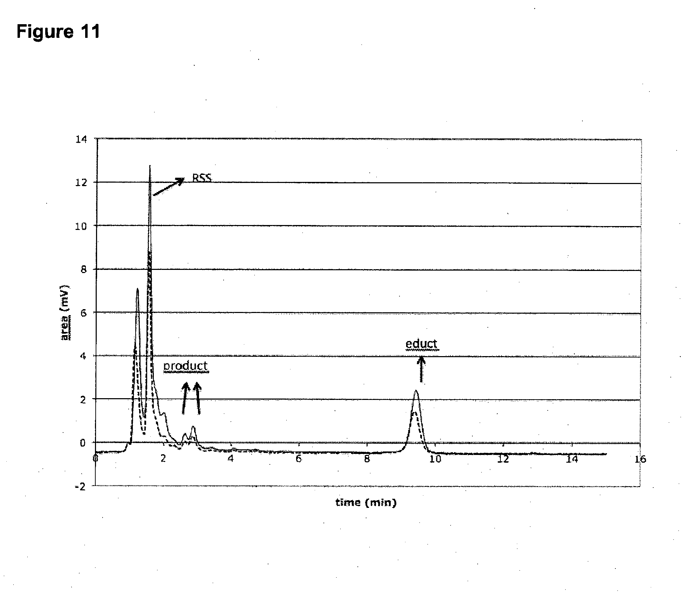

Further experiments using a non-membrane transferrable substrate, abietic acid, were conducted. (Figs. 9-12) and it was shown that a product occurred at the expected retention time if protein expression was induced with 1 mM IPTG.

A similar approach starting from amplification of the gene by PCR to successful HPLC activity measurements was performed for the human enzyme CYP3A4.

We succeeded to display the P450 enzymes CYP106A2 and CYP3A on the surface of *E*. *coli* in a functional form by using the autodisplay system. Functional expression was achieved by a one step procedure after induction of protein expression. Without wishing to by bound by theory, the prosthetic group was incorporated during transport in the periplasm, and the folded protein was translocated to the cell surface by the aid of the Omp85 pathway.

### Example 2

This example refers to the role of TolC present in the outer membrane of *E*. *coli* cells displaying CYP106A2 on the surface. TolC is involved in porphyrin transport across the cell membrane [26].

Functional expression was determined by the CYP106A2-dependent conversion of 11-deoxycorticosterone to 15β-deoxycorticosterone, employing the experimental condition as described in Example 1.

The strain BL21 (DE3) employed in Example 1 expresses TolC on the outer membrane. Therefore, this strain has the capability to transport porphyrins, including P450, to the outer membrane surface.

The strain JW5503-1 (DE3) is a TolC defective mutant, with a reduced capability of transporting porphyrins (including P450) onto the outer membrane surface. JW5503-1 was obtained from the Keio collection distributed by *Coli* Genetic Stock Center at Yale University.

Figure 15 indicates that CYP106A2 is expressed by pET-CYP13 to the same extent on the surface on TolC negative strain JW5503-1 (DE3) and on TolC postive BL21 (DE3) *E*. *coli* cells.

Figure 16 shows HPLC chromatograms of CYP106A2 conversion of 11-deoxycorticosterone to 15β-deoxycorticosterone. By comparison of Figure 16C and B, it can be seen that the amount of 15β-deoxycorticosterone decreased when CYP106A2 was expressed in the *E*. *coli* strain lacking the TolC channel protein.

It is concluded that TolC can provide porphyrins, in particular P450, on the cell surface, so that the porphyrins can be introduced into a recombinant surface-displayed polypeptide of the present invention, such as CYP106A.

Figure 16 indicates that there is still a CYP106A activity in the absence of TolC. Thus other transporters different from TolC may be present in the outer membrane so that porphyrins, in particular P450, can be provided on the cell surface so that the prosthetic group can be introduced into a recombinant surface-displayed polypeptide of the present invention, such as CYP106A.

### Example 3

### N-demethylation of the antidepressant imipramine into its active metabolite, desipramine.

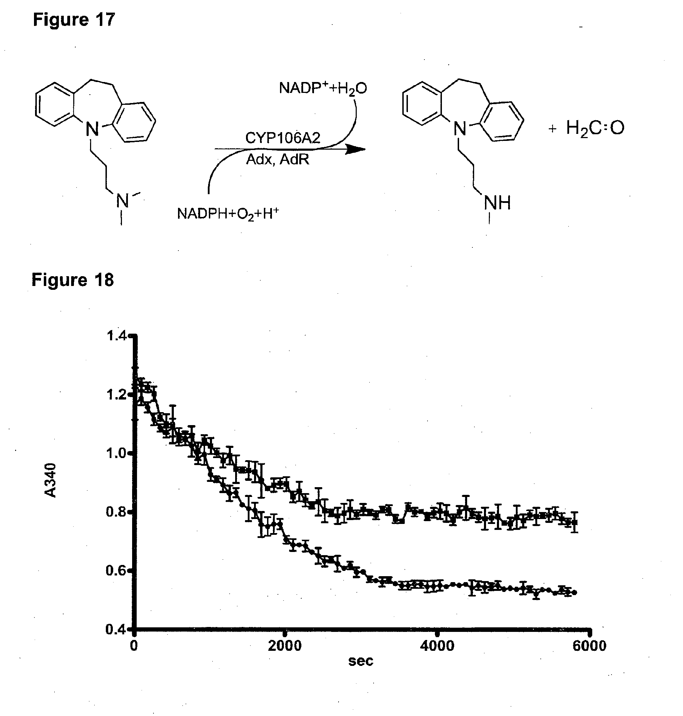

The reaction as illustrated by Figure 17 is catalyzed by CYP106A2 displayed on the surface of *E*. *coli* cells. The cells were prepared as described in Example 1. Redox equivalents are transferred from NADPH via the proteins AdR and Adx to the converting enzyme.

The reaction mixture contained in a total volume of 0.2 mL Hepes buffer (50 mM, 0.05 % Tween20, pH 7.4), imipramine (2.5 mM), Adx (5 mM), AdR (0.5 mM), NADPH (200 mM) and cells of *E*. *coli* BL21(DE3) or BL21(DE3) pETCYP13 corresponding to an OD₅₇₈= 2.5.

Figure 18 shows the kinetics of NADPH consumption of cells by *E*. *coli* BL21(DE3) pETCYP13 expressing CYP106A2 cells and control BL21(DE3) cells in the presence of imipramine, as indicated above. The by-product formalin (cf. Figure 17) has been photometrically identified to be produced by the cells displaying CYP106A2, but not in the control cells (data not shown). Thus, the difference of CYP106A2 expressing cells and control cells in NADPH consumption indicates conversion of imipramine into desipramine.

### References

1. Kaur J, Sharma R (2006) Crit Rev Biotechnol 26, 165-199.
2. Schoemaker HE, Mink D, Wubbolts MG (2003) Science 299, 1694-1697.
3. Rubin-Pitel SB, Zhao H (2006) Comb Chem High Throughput Screen 9, 247-257.
4. Bornscheuer UT (2005) Adv Biochem Engin Biotechnol 100, 181-203.
5. Bernhardt R (2006) J Biotechnol 124, 128-145.
6. Li Y, Drummond DA, Sawayama AM, Snow CD, Bloom JD, Arnold FH (2007) Nat Biotechnol 9, 1051-1056.
7. Hannemann F, Virus C, Bernhardt R (2006) J Biotechnol 124, 72-81.
8. Guengerich FP (2008) Chem Res Toxicol 21, 70-83.
9. Jose J, Meyer TF (2007) Microbiol Mol Biol 71, 600-619.
10. Jose J (2006) Appl Microbiol Biotechnol 69, 607-614.
11. Jose J, Bernhardt R, Hannemann F (2001) ChemBioChem 2, 695-701.
12. Jose J, Bernhardt R, Hannemann F (2002) J Biotechnol 95, 257-268.
13. Jose J, Jähnig F, Meyer TF (1995) Mol Microbiol 18, 378-380.
14. Pohlner J, Halter R, Beyreuther K, Meyer TF (1987) Nature 325, 458-462.
15. Maurer J, Jose J, Meyer TF (1997) J Bacteriol 179, 794-804.
16. Jose J, Handel S (2003) ChemBioChem 4, 396-405.
17. Jose J, von Schwichow S (2004) Anal Biochem 331, 267-274.
18. Klauser T, Pohlner J, Meyer TF (1990) Embo J 9, 1991-1999.
19. Jose J, Krämer J, Klauser T, Pohlner J, Meyer TF (1996) Gene 178, 107-110.
20. Jose J, Zangen D (2005) Biochem Biophys Res Commun 333, 1218-1226.
21. Jose J, von Schwichow S (2004) ChemBioChem 5, 491-499.
22. Hannemann F, Bernhardt R, Jose J (2007) J Enz Inhib Med Chem 22, 570-576.
23. Hantke, K., 1981. Regulation of ferric iron transport in Escherichia coli K12: isolation of a constitutive mutant. Mol. Gen. Genet. 182, 288-292.
24. Henderson I et al., 2004. Type V protein secretion pathway: the autotransporter story. Microbiology and Molecular Biology Reviews, 68(4), 692-744
25. Schultheiss, E., Paar, C., Schwab, H., Jose, J., 2002. Functional esterase surface display by the autotransporter pathway in Escherichia coli. J. Mol. Catal., B Enzym. 18, 89-97.
26. Ryoko Tatsumi, Masaaki Wachi (2008), TolC-Dependent Exclusion of Porphyrins in Escherichia coli. J Bacteriol. 190(18):6228-6233.Published online 2008 July 18.

## Claims

1. A method for displaying a recombinant polypeptide containing a prosthetic group on the surface of a host cell, wherein the prosthetic group comprises a metal porphyrin or a flavin, said method comprising the steps:
(a) providing a host cell transformed with a nucleic acid fusion operatively linked with an expression control sequence, said nucleic acid fusion comprising:
(i) a portion encoding a signal peptide,
(ii) a portion encoding the recombinant polypeptide to be displayed,
(iii) a portion encoding a transmembrane linker, and
(iv) a portion encoding the transporter domain of an autotransporter,
and
(b) culturing the host cell under conditions wherein the nucleic acid fusion is expressed and the expression product comprising the recombinant polypeptide containing the prosthetic group is displayed on the surface of the host cell,
wherein the prosthetic group is transported to the cell surface independently from the expression product comprising the recombinant polypeptide.

2. The method according to claim 1, wherein the nucleic acid fusion further comprises a portion encoding a protease recognition site.

3. The method according to claim 1 or 2 wherein the polypeptide comprising the metal porphyrin is selected from hemoproteins, P450 enzymes, P450 reductases, cytochromes, and monooxygenases.

4. The method according to any one of the preceding claims, wherein the prosthetic group being a metal porphyrin is transported to the cell surface by a TolC-dependent mechanism.

5. The method according to claim 1 or 2 wherein the polypeptide is selected from flavoproteins and preferably comprises a flavin selected from FAD and FMN.

6. The method according to any one of the preceding claims wherein the host cell is a bacterium, particularly a Gram-negative bacterium, more particularly an enterobacterium, e.g. *E*. *coli.*

7. The method according to any one of the preceding claims wherein the transporter domain of the autotransporter is selected from Ssp, Ssp-h1, Ssp-h2, PspA, PspB, Ssa1, SphB1, AspA/NalP, VacA, AIDA-I, IcsA, MisL, TibA, Ag43, ShdA, AutA, Tsh, SepA, EspC, EspP, Pet, Pic, SigA, Sat, Vat, EpeA, EatA, Espl, EaaA, EaaC, Pertactin, BrkA, Tef, Vag8, PmpD, Pmp20, Pmp21, IgA1 protease, App, Hap, rOmpA, rOmpB, ApeE, EstA, Lip-1, McaP, BabA, SabA, AlpA, Aae, NanB, and variants having at least 90% sequence identity thereto, and wherein the transporter domain of the autotransporter preferably is the *E*. *coli* AIDA-1 protein or a variant thereof having at least 90% sequence identity to the *E. coli* AIDA-I protein.

8. The method according to any one of the preceding claims comprising (c) preparing a membrane preparation from the cell obtained in step (b).

9. Host cell displaying a recombinant polypeptide on the surface thereof wherein the recombinant polypeptide contains a prosthetic group comprising a metal porphyrin or a flavin, and wherein an expression product comprising the recombinant polypeptide comprises
(I) a portion comprising the recombinant polypeptide to be displayed,
(II) a portion comprising a transmembrane linker, and
(III) a portion comprising the transporter domain of an autotransporter, wherein the host cell recombinantly expresses a transport protein capable of transporting the prosthetic group to the host cell surface, and wherein the prosthetic group is transported to the host cell surface by the transport protein independently from the expression product comprising the recombinant polypeptide.

10. The host cell of claim 9, wherein the expression product further comprises a portion comprising a protease recognition site.

11. The host cell of claim 9 or 10 wherein the recombinant polypeptide is displayed by the transporter domain of an autotransporter.

12. The host cell according to any one of the claims 9 to 11, wherein the polypeptide comprising the metal porphyrin is selected from hemoproteins, P450 enzymes, P450 reductases, cytochromes, and monooxygenases.

13. The host cell according to any one of the claims 9 to 12, wherein the prosthetic group being a metal porphyrin is transported to the cell surface by a TolC-dependent mechanism.

14. The host cell according to any one of the claims 9 to 11, wherein the polypeptide is selected from flavoproteins and preferably comprises a flavin selected from FAD and FMN.

15. The host cell according to any one of the claims 9 to 14 wherein the host cell is a bacterium, particularly a Gram-negative bacterium, more particularly an enterobacterium, e.g. *E*. *coli.*

16. Use of a cell of any one of the claims 9 to 15 for a chemical synthesis procedure, preferably for the synthesis of organic substances selected from enzyme substrates, drugs, hormones, starting materials and intermediates for synthesis procedures and chiral substances.

17. Use of a cell of any one of the claims 9 to 15
(a) for a directed evolution procedure,
(b) as an assay system for a screening procedure, e.g. for identifying modulators of metal porphyrin containing enzymes,
(c) as a system for toxicity monitoring, or/and
(d) as a system for degrading toxic substances.

## Patentansprüche

1. Verfahren zur Präsentation eines rekombinanten Polypeptids enthaltend eine prosthetische Gruppe auf der Oberfläche einer Wirtszelle, wobei die prosthetische Gruppe ein Metallporphyrin oder ein Flavin enthält, wobei das Verfahren die Schritte umfasst:
(a) Bereitstellen einer Wirtszelle transformiert mit einer Nukleinsäurefusion operativ verbunden mit einer Expressionskontrollsequenz, wobei die Nukleinsäurefusion umfasst:
(i) einen Anteil kodierend ein Signalpeptid,
(ii) einen Anteil kodierend das rekombinante Polypeptid, welches präsentiert werden soll,
(iii) einen Anteil kodierend einen Transmembranlinker, und
(iv) einen Anteil kodierend die Transporterdomäne eines Autotransporters, und
(b) Kultivieren der Wirtszelle unter Bedingungen, unter denen die Nukleinsäurefusion exprimiert wird und das Expressionsprodukt umfassend das rekombinante Polypeptid enthaltend die prosthetische Gruppe auf der Oberfläche der Wirtszelle präsentiert wird,
wobei die prosthetische Gruppe unabhängig vom Expressionsprodukt umfassend das rekombinante Polypeptid auf die Zelloberfläche transportiert wird.

2. Verfahren gemäß Anspruch 1, wobei die Nukleinsäurefusion weiterhin einen Anteil kodierend eine Proteaseerkennungsstelle umfasst.

3. Verfahren gemäß Anspruch 1 oder 2, wobei das Polypeptid umfassend das Metallporphyrin ausgewählt wird aus Hämproteinen, P450-Enzymen, P450-Reduktasen, Cytochromen und Monooxidasen.

4. Verfahren gemäß einem der vorhergehenden Ansprüche, wobei die prosthethische Gruppe, welche ein Metallporphyrin ist, durch einen TolCabhängigen Mechanismus auf die Zelloberfläche transportiert wird.

5. Verfahren gemäß Anspruch 1 oder 2, wobei das Polypeptid ausgewählt wird aus Flavoproteinen und vorzugsweise ein Flavin ausgewählt aus FAD und FMN umfasst.

6. Verfahren gemäß einem der vorhergehenden Ansprüche, wobei die Wirtszelle ein Bakterium, vorzugsweise ein gramnegatives Bakterium, stärker bevorzugt ein Enterobakterium, z. B. *E*. *coli,* ist.

7. Verfahren gemäß einem der vorhergehenden Ansprüche, wobei die Transporterdomäne des Autotransporters ausgewählt wird aus Ssp, Ssp-h1, Ssp-h2, PspA, PspB, Ssa1, SphB1, AspA/NalP, VacA, AIDA-I, IcsA, MisL, TibA, Ag43, ShdA, AutA, Tsh, SepA, EspC, EspP, Pet, Pic, SigA, Sat, Vat, EpeA, EatA, Espl, EaaA, EaaC, Pertactin, BrkA, Tef, Vag8, PmpD, Pmp20, Pmp21, IgA1-Protease, App, Hap, rOmpA, rOmpB, ApeE, EstA, Lip-1, McaP, BabA, SabA, AlpA, Aae, NanB und Varianten davon, welche wenigstens 90 % Sequenzidentität dazu haben, und wobei die Transporterdomäne des Autotransporters vorzugsweise das E. *coli* AIDA-I Protein oder eine Variante davon mit wenigstens 90 % Sequenzidentität zum *E*. *coli* AIDA-I Protein ist.

8. Verfahren gemäß einem der vorhergehenden Ansprüche, umfassend (c) Herstellen einer Membranpräparation aus der Zelle, welche in Schritt (b) erhalten wird.

9. Wirtszelle präsentierend ein rekombinantes Polypeptid auf der Oberfläche, wobei das rekombinante Polypeptid eine prosthetische Gruppe umfassend ein Metallporphyrin oder ein Flavin enthält, und wobei ein Expressionsprodukt umfassend das rekombinante Polypeptid umfasst:
(I) einen Anteil umfassend das rekombinante Polypeptid, welches präsentiert werden soll,
(II) einen Anteil umfassend einen Transmembranlinker, und
(III) einen Anteil umfassend die Transporterdomäne eines Autotransporters,
wobei die Wirtszelle ein Transportprotein rekombinant exprimiert, welches die prosthetische Gruppe auf die Oberfläche der Wirtszelle transportieren kann, und wobei die prosthetische Gruppe durch das Transportprotein unabhängig vom Expressionsprodukt umfassend das rekombinante Polypeptid auf die Zelloberfläche transportiert wird.

10. Wirtszelle gemäß Anspruch 9, wobei das Expressionsprodukt weiterhin einen Anteil kodierend eine Proteaseerkennungsstelle umfasst.

11. Wirtszelle gemäß Anspruch 9 oder 10, wobei das rekombinante Polypeptid durch die Transporterdomäne eines Autotransporters präsentiert wird.

12. Wirtszelle gemäß einem der Ansprüche 9 bis 11, wobei das Polypeptid umfassend das Metallporphyrin ausgewählt wird aus Hämproteinen, P450-Enzymen, P450-Reduktasen, Cytochromen und Monooxidasen.

13. Wirtszelle gemäß einem der Ansprüche 9 bis 12, wobei die prosthethische Gruppe, welche ein Metallporphyrin ist, durch einen TolC-abhängigen Mechanismus auf die Zelloberfläche transportiert wird.

14. Wirtszelle gemäß einem der Ansprüche 9 bis 11, wobei das Polypeptid ausgewählt wird aus Flavoproteinen und vorzugsweise ein Flavin ausgewählt aus FAD und FMN umfasst.

15. Wirtszelle gemäß einem der Ansprüche 9 bis 14, wobei die Wirtszelle ein Bakterium, vorzugsweise ein gramnegatives Bakterium, stärker bevorzugt ein Enterobakterium, z. B. *E*. *coli,* ist.

16. Verwendung einer Zelle gemäß einem der Ansprüche 9 bis 15 für ein chemisches Syntheseverfahren, vorzugsweise für die Synthese von organischen Substanzen ausgewählt aus Enzymsubstraten, Wirkstoffen, Hormonen, Ausgangsmaterialien und Zwischenprodukten für Syntheseverfahren und chiralen Substanzen.

17. Verwendung einer Zelle gemäß einem der Ansprüche 9 bis 15
(a) für ein gerichtetes Evolutionsverfahren,
(b) als ein Testsystem für ein Screeningverfahren, z. B. zur Identifizierung von Modulatoren von Metallporphyrin enthaltenden Enzymen,
(c) als ein System zur Toxizitätsüberwachung, oder/und
(d) als ein System zum Abbau toxischer Substanzen.

## Revendications

1. Procédé d'affichage d'un polypeptide recombinant contenant un groupe prosthétique à la surface d'une cellule hôte, dans lequel le groupe prosthétique comprend une porphyrine métallique ou une flavine, ledit procédé comprenant les étapes de :
(a) fournir une cellule hôte transformée avec un acide nucléique de fusion lié de façon opérationnelle à une séquence de contrôle d'expression, ledit acide nucléique de fusion comprenant :
(i) une partie codant un peptide signal,
(ii) une partie codant un polypeptide recombinant qui doit être affiché,
(iii) une partie codant pour un linker transmembranaire, et
(iv) une partie codant le domaine transporteur d'un auto-transporteur, et
(b) cultiver la cellule hôte dans des conditions telles que l'acide nucléique de fusion est exprimé et le produit d'expression comprenant le polypeptide recombinant contenant le groupe prosthétique est affiché à la surface de la cellule hôte,
dans lequel le groupe prosthétique est transporté à la surface de la cellule indépendamment du produit d'expression comprenant le polypeptide recombinant.

2. Procédé selon la revendication 1, dans lequel l'acide nucléique de fusion comprend de plus une partie codant pour une protéase de reconnaissance de site.

3. Procédé selon la revendication 1 ou 2, dans lequel le polypeptide comprenant la porphyrine métallique est choisi parmi les hémoprotéines, les enzymes P450, les réductases P450, des cytochromes et des mono-oxygénases.

4. Procédé selon l'une quelconque des revendications précédentes, dans lequel le groupe prosthétique étant une porphyrine métallique est transportée à la surface de la cellule par un mécanisme TolC dépendant.

5. Procédé selon la revendication 1 ou 2, dans lequel le polypeptide est choisi parmi les flavoprotéines et de préférence comprend une flavine choisie parmi FAD ou FMN.

6. Procédé selon l'une quelconque des revendications précédentes, dans lequel la cellule hôte est une bactérie, en particulier une bactérie Gram négatif, plus particulièrement une entérobactérie, par exemple, *E. coli.*

7. Procédé selon l'une quelconque des revendications précédentes, dans lequel le domaine transporteur de l'auto-transporteur est choisi dans Ssp, Ssp-h1, Ssp-h2, PspA, PspB, Ssa1, SphB1, AspA / NalP, VacA, AIDA-I, IcsA, MisL, TibA, Ag43, ShdA, AutA, Tsh, SepA, EspC, EspP, Pet, Pic, SigA, Sat, Vat, EpeA, EatA, Espl, EaaA, EaaC, Pertactin, BrkA, Tef, Vag8, PmpD, Pmp20, Pmp21, IgA1 protéase, App, Hap, rOmpa, rOmpB, ApeE, EstA, Lip-1, McaP, BabA, SabA, AlpA, Aae, NanB, et leurs variantes ayant au moins 90% d'identité en séquence avec ceux-ci, et dans lequel the domaine transporteur de l'auto-transporteur de préférence est la protéine E. *coli* AIDA-I ou une variante de celle-ci ayant au moins 90% d'identité en séquence avec la protéine *E.coli* AIDA-I.

8. Procédé selon l'une quelconque des revendications précédentes comprenant (c) préparer une préparation membranaire à partir d'une cellule obtenue à l'étape (b).

9. Cellule hôte affichant un polypeptide recombinant à la surface de celle-ci, dans lequel le polypeptide recombinant contient un groupe prosthétique comprenant une porphyrine métallique ou une flavine, et dans lequel un produit d'expression comprenant un polypeptide recombinant comprend
(I) une partie comprenant un polypeptide recombinant destiné à être affiché
(II) une partie comprenant un linker transmembranaire, et
(III) une partie comprenant un domaine transporteur et l'auto-transporteur,
dans laquelle la cellule hôte exprime de façon recombinante une protéine de transport capable de transporter le groupe prosthétique à la surface de la cellule hôte, et dans laquelle le groupe prosthétique est transporté à la surface de la cellule par une protéine de transport indépendamment du produit d'expression comprenant le polypeptide recombinant.

10. Cellule hôte selon la revendication 9, dans laquelle le produit d'expression comprend en plus une partie comprenant un site de reconnaissance de protéases.

11. Cellule hôte selon la revendication 9 ou 10, dans laquelle le polypeptide recombinant est affiché par le domaine transporteur de l'auto-transporteur.

12. Cellule hôte selon l'une quelconque des revendications 9 à 11, dans laquelle le polypeptide comprenant une porphyrine métallique est choisi parmi les hémoprotéines, les enzymes P450, les réductases P450, des cytochromes et des mono-oxygénases.

13. Cellule hôte selon l'une quelconque des revendications 9 à 12, dans laquelle le groupe prosthétique étant une porphyrine métallique est transportée à la surface de la cellule par un mécanisme TolC dépendant.

14. Cellule hôte selon l'une quelconque des revendications 9 à 11, dans laquelle le polypeptide est choisi parmi les flavoprotéines et de préférence comprend une flavine choisie parmi FAD ou FMN.

15. Cellule hôte selon l'une quelconque des revendications 9 à 14, dans laquelle le polypeptide est une bactérie, en particulier une bactérie Gram négatif, plus particulièrement une entérobactérie, par exemple *E*. *coli.*

16. Utilisation d'une cellule selon l'une quelconque des revendications 9 à 15 pour un procédé de synthèse chimique, de préférence pour la synthèse de substances organiques choisies parmi les substrats enzymatiques, les médicaments, les hormones, les matières premières et les intermédiaires de procédés de synthèse et les substances chirales.

17. Utilisation d'une cellule selon l'une quelconque des revendications 9 à 15
(a) pour une méthode d'évolution dirigée,
(b) en tant que système d'essai pour un procédé de criblage, par exemple pour l'identification de modulateurs de porphyrines métalliques contenant des enzymes,
(c) en tant que système pour contrôler la toxicité, ou/et
(d) en tant que système pour dégrader les substances toxiques.


### SEQUENCE LISTING

<110> Autodisplay Biotech GmbH
<120> p450 surface display
<130> 49058PWO
<150> EP10179060.8
   <151> 2010-09-23
<160> 14
<170> BiSSAP 1.0
<210> 1
   <211> 1242
   <212> DNA
   <213> Artificial Sequence
<220>
   <221> source
   <222> 1..1242
   <223> /mol_type="DNA" /note="CYP106A2 insert in plasmid pET-CYP13" /organism="Artificial Sequence"
<400> 1
   ctcgagatgg aagaagttat tgcagtaaaa gaaattacta ggtttaaaac aaggacggag      60
   gaatttagcc cgtacgcttg gtgtaaaagg atgttagaaa atgaccctgt gagttatcac     120
   gaaggaacgg atacgtggaa tgtctttaaa tatgaagatg tgaagcgggt tctcagtgat     180
   tataaacatt tttcaagtgt tcggaaacgg acgacgattt cagttggaac ggatagtgag     240
   gaaggttctg tgcctgaaaa gatccaaatc actgaatcgg atccacctga tcatagaaaa     300
   cgccgttcac tgctggcagc agcattcaca cctagaagtc ttcaaaactg ggaacctcgc     360
   attcaggaaa ttgcagatga attgattgga caaatggatg gtggaacgga aatcgatatt     420
   gtggcatcat tggcgagtcc gcttccgatc attgtcatgg ccgatttgat gggggttccc     480
   tcgaaagatc gtttattgtt taagaaatgg gtggatacct tatttcttcc ttttgataga     540
   gaaaagcaag aagaagtaga taaattgaag caagttgcag caaaagaata ctatcagtat     600
   ttgtatccga ttgttgtgca aaaacgattg aacccggcgg atgatatcat ctcagatcta     660
   ttgaagtcgg aagtggatgg ggaaatgttt acggatgatg aggttgtccg gacgaccatg     720
   ctgattttag gtgcaggagt cgagacaacc agtcatttat tggccaatag cttttattcg     780
   ctgctatatg atgacaaaga agtttatcaa gagttacatg aaaacctgga tttagttccg     840
   caggcggtcg aagaaatgct ccgtttccga ttcaatctta ttaaattgga tcgcactgta     900
   aaggaagata acgatctatt gggagtggaa ttgaaagaag gggatagcgt ggttgtttgg     960
   atgagtgcag ctaatatgga cgaagagatg tttgaagacc ccttcacact taatatccac    1020
   cgccctaata ataagaaaca tctcacattc ggtaatggcc ctcatttctg cctcggagca    1080
   ccgctagcca ggctggaagc gaagattgcg cttactgcat tcctgaagaa attcaagcat    1140
   attgaagcgg tgccatcgtt ccagttagaa gagaatctta ccgattcagc gaccggtcaa    1200
   actttgacct cactaccgct taaggcaagc cgcatgggta cc                       1242
<210> 2
   <211> 414
   <212> PRT
   <213> Artificial Sequence
<220>
   <221> SOURCE
   <222> 1..414
   <223> /mol_type="protein" /note="CYP106A2 insert in plasmid pET-CYP13" /organism="Artificial Sequence"
<400> 2
   Leu Glu Met Glu Glu Val Ile Ala Val Lys Glu Ile Thr Arg Phe Lys
   1               5                   10                  15
   Thr Arg Thr Glu Glu Phe Ser Pro Tyr Ala Trp Cys Lys Arg Met Leu
               20                  25                  30
   Glu Asn Asp Pro Val Ser Tyr His Glu Gly Thr Asp Thr Trp Asn Val
           35                  40                  45
   Phe Lys Tyr Glu Asp Val Lys Arg Val Leu Ser Asp Tyr Lys His Phe
       50                  55                  60
   Ser Ser Val Arg Lys Arg Thr Thr Ile Ser Val Gly Thr Asp Ser Glu
   65                  70                  75                  80
   Glu Gly Ser Val Pro Glu Lys Ile Gln Ile Thr Glu Ser Asp Pro Pro
                   85                  90                  95
   Asp His Arg Lys Arg Arg Ser Leu Leu Ala Ala Ala Phe Thr Pro Arg
               100                 105                 110
   Ser Leu Gln Asn Trp Glu Pro Arg Ile Gln Glu Ile Ala Asp Glu Leu
           115                 120                 125
   Ile Gly Gln Met Asp Gly Gly Thr Glu Ile Asp Ile Val Ala Ser Leu
       130                 135                 140
   Ala Ser Pro Leu Pro Ile Ile Val Met Ala Asp Leu Met Gly Val Pro
   145                 150                 155                 160
   Ser Lys Asp Arg Leu Leu Phe Lys Lys Trp Val Asp Thr Leu Phe Leu
                   165                 170                 175
   Pro Phe Asp Arg Glu Lys Gln Glu Glu Val Asp Lys Leu Lys Gln Val
               180                 185                 190
   Ala Ala Lys Glu Tyr Tyr Gln Tyr Leu Tyr Pro Ile Val Val Gln Lys
           195                 200                 205
   Arg Leu Asn Pro Ala Asp Asp Ile Ile Ser Asp Leu Leu Lys Ser Glu
       210                 215                 220
   Val Asp Gly Glu Met Phe Thr Asp Asp Glu Val Val Arg Thr Thr Met
   225                 230                 235                 240
   Leu Ile Leu Gly Ala Gly Val Glu Thr Thr Ser His Leu Leu Ala Asn
                   245                 250                 255
   Ser Phe Tyr Ser Leu Leu Tyr Asp Asp Lys Glu Val Tyr Gln Glu Leu
               260                 265                 270
   His Glu Asn Leu Asp Leu Val Pro Gln Ala Val Glu Glu Met Leu Arg
           275                 280                 285
   Phe Arg Phe Asn Leu Ile Lys Leu Asp Arg Thr Val Lys Glu Asp Asn
       290                 295                 300
   Asp Leu Leu Gly Val Glu Leu Lys Glu Gly Asp Ser Val Val Val Trp
   305                 310                 315                 320
   Met Ser Ala Ala Asn Met Asp Glu Glu Met Phe Glu Asp Pro Phe Thr
                   325                 330                 335
   Leu Asn Ile His Arg Pro Asn Asn Lys Lys His Leu Thr Phe Gly Asn
               340                 345                 350
   Gly Pro His Phe Cys Leu Gly Ala Pro Leu Ala Arg Leu Glu Ala Lys
           355                 360                 365
   Ile Ala Leu Thr Ala Phe Leu Lys Lys Phe Lys His Ile Glu Ala Val
       370                 375                 380
   Pro Ser Phe Gln Leu Glu Glu Asn Leu Thr Asp Ser Ala Thr Gly Gln
   385                 390                 395                 400
   Thr Leu Thr Ser Leu Pro Leu Lys Ala Ser Arg Met Gly Thr
                   405                 410
<210> 3
   <211> 810
   <212> PRT
   <213> Escherichia coli
<220>
   <221> SOURCE
   <222> 1..810
   <223> /mol_type="protein" /note=" Outer membrane protein assembly factor, Omp85 homologue" /organism="Escherichia coli"
<400> 3
   Met Ala Met Lys Lys Leu Leu Ile Ala Ser Leu Leu Phe Ser Ser Ala
   1               5                   10                  15
   Thr Val Tyr Gly Ala Glu Gly Phe Val Val Lys Asp Ile His Phe Glu
               20                  25                  30
   Gly Leu Gln Arg Val Ala Val Gly Ala Ala Leu Leu Ser Met Pro Val
           35                  40                  45
   Arg Thr Gly Asp Thr Val Asn Asp Glu Asp Ile Ser Asn Thr Ile Arg
       50                  55                  60
   Ala Leu Phe Ala Thr Gly Asn Phe Glu Asp Val Arg Val Leu Arg Asp
   65                  70                  75                  80
   Gly Asp Thr Leu Leu Val Gln Val Lys Glu Arg Pro Thr Ile Ala Ser
                   85                  90                  95
   Ile Thr Phe Ser Gly Asn Lys Ser Val Lys Asp Asp Met Leu Lys Gln
               100                 105                 110
   Asn Leu Glu Ala Ser Gly Val Arg Val Gly Glu Ser Leu Asp Arg Thr
           115                 120                 125
   Thr Ile Ala Asp Ile Glu Lys Gly Leu Glu Asp Phe Tyr Tyr Ser Val
       130                 135                 140
   Gly Lys Tyr Ser Ala Ser Val Lys Ala Val Val Thr Pro Leu Pro Arg
   145                 150                 155                 160
   Asn Arg Val Asp Leu Lys Leu Val Phe Gln Glu Gly Val Ser Ala Glu
                   165                 170                 175
   Ile Gln Gln Ile Asn Ile Val Gly Asn His Ala Phe Thr Thr Asp Glu
               180                 185                 190
   Leu Ile Ser His Phe Gln Leu Arg Asp Glu Val Pro Trp Trp Asn Val
           195                 200                 205
   Val Gly Asp Arg Lys Tyr Gln Lys Gln Lys Leu Ala Gly Asp Leu Glu
       210                 215                 220
   Thr Leu Arg Ser Tyr Tyr Leu Asp Arg Gly Tyr Ala Arg Phe Asn Ile
   225                 230                 235                 240
   Asp Ser Thr Gln Val Ser Leu Thr Pro Asp Lys Lys Gly Ile Tyr Val
                   245                 250                 255
   Thr Val Asn Ile Thr Glu Gly Asp Gln Tyr Lys Leu Ser Gly Val Glu
               260                 265                 270
   Val Ser Gly Asn Leu Ala Gly His Ser Ala Glu Ile Glu Gln Leu Thr
           275                 280                 285
   Lys Ile Glu Pro Gly Glu Leu Tyr Asn Gly Thr Lys Val Thr Lys Met
       290                 295                 300
   Glu Asp Asp Ile Lys Lys Leu Leu Gly Arg Tyr Gly Tyr Ala Tyr Pro
   305                 310                 315                 320
   Arg Val Gln Ser Met Pro Glu Ile Asn Asp Ala Asp Lys Thr Val Lys
                   325                 330                 335
   Leu Arg Val Asn Val Asp Ala Gly Asn Arg Phe Tyr Val Arg Lys Ile
               340                 345                 350
   Arg Phe Glu Gly Asn Asp Thr Ser Lys Asp Ala Val Leu Arg Arg Glu
               355                     360                     365
   Met Arg Gln Met Glu Gly Ala Trp Leu Gly Ser Asp Leu Val Asp Gln
           370                     375                     380
   Gly Lys Glu Arg Leu Asn Arg Leu Gly Phe Phe Glu Thr Val Asp Thr
   385                     390                     395                     400
   Asp Thr Gln Arg Val Pro Gly Ser Pro Asp Gln Val Asp Val Val Tyr
                   405                     410                     415
   Lys Val Lys Glu Arg Asn Thr Gly Ser Phe Asn Phe Gly Ile Gly Tyr
               420                     425                     430
   Gly Thr Glu Ser Gly Val Ser Phe Gln Ala Gly Val Gln Gln Asp Asn
           435                     440                     445
   Trp Leu Gly Thr Gly Tyr Ala Val Gly Ile Asn Gly Thr Lys Asn Asp
       450                     455                     460
   Tyr Gln Thr Tyr Ala Glu Leu Ser Val Thr Asn Pro Tyr Phe Thr Val
   465                     470                     475                     480
   Asp Gly Val Ser Leu Gly Gly Arg Leu Phe Tyr Asn Asp Phe Gln Ala
                   485                     490                     495
   Asp Asp Ala Asp Leu Ser Asp Tyr Thr Asn Lys Ser Tyr Gly Thr Asp
               500                     505                     510
   Val Thr Leu Gly Phe Pro Ile Asn Glu Tyr Asn Ser Leu Arg Ala Gly
           515                     520                     525
   Leu Gly Tyr Val His Asn Ser Leu Ser Asn Met Gln Pro Gln Val Ala
       530                     535                     540
   Met Trp Arg Tyr Leu Tyr Ser Met Gly Glu His Pro Ser Thr Ser Asp
   545                     550                     555                     560
   Gln Asp Asn Ser Phe Lys Thr Asp Asp Phe Thr Phe Asn Tyr Gly Trp
                   565                     570                     575
   Thr Tyr Asn Lys Leu Asp Arg Gly Tyr Phe Pro Thr Asp Gly Ser Arg
               580                     585                     590
   Val Asn Leu Thr Gly Lys Val Thr Ile Pro Gly Ser Asp Asn Glu Tyr
           595                     600                     605
   Tyr Lys Val Thr Leu Asp Thr Ala Thr Tyr Val Pro Ile Asp Asp Asp
       610                     615                     620
   His Lys Trp Val Val Leu Gly Arg Thr Arg Trp Gly Tyr Gly Asp Gly
   625                     630                     635                     640
   Leu Gly Gly Lys Glu Met Pro Phe Tyr Glu Asn Phe Tyr Ala Gly Gly
                   645                     650                     655
   Ser Ser Thr Val Arg Gly Phe Gln Ser Asn Thr Ile Gly Pro Lys Ala
               660                     665                     670
   Val Tyr Phe Pro His Gln Ala Ser Asn Tyr Asp Pro Asp Tyr Asp Tyr
           675                     680                     685
   Glu Cys Ala Thr Gln Asp Gly Ala Lys Asp Leu Cys Lys Ser Asp Asp
       690                     695                     700
   Ala Val Gly Gly Asn Ala Met Ala Val Ala Ser Leu Glu Phe Ile Thr
   705                     710                     715                     720
   Pro Thr Pro Phe Ile Ser Asp Lys Tyr Ala Asn Ser Val Arg Thr Ser
                   725                     730                     735
   Phe Phe Trp Asp Met Gly Thr Val Trp Asp Thr Asn Trp Asp Ser Ser
               740                     745                     750
   Gln Tyr Ser Gly Tyr Pro Asp Tyr Ser Asp Pro Ser Asn Ile Arg Met
           755                     760                     765
   Ser Ala Gly Ile Ala Leu Gln Trp Met Ser Pro Leu Gly Pro Leu Val
       770                     775                     780
   Phe Ser Tyr Ala Gln Pro Phe Lys Lys Tyr Asp Gly Asp Lys Ala Glu
   785                     790                     795                     800
   Gln Phe Gln Phe Asn Ile Gly Lys Thr Trp
                   805                     810
<210> 4
   <211> 810
   <212> PRT
   <213> Escherichia coli
<220>
   <221> SOURCE
   <222> 1..810
   <223> /mol_type="protein" /note="Outer membrane protein assembly factor, Omp85 homologue" /organism="Escherichia coli"
<400> 4
   Met Ala Met Lys Lys Leu Leu Ile Ala Ser Leu Leu Phe Ser Ser Ala
   1               5                   10                  15
   Thr Val Tyr Gly Ala Glu Gly Phe Val Val Lys Asp Ile His Phe Glu
               20                  25                  30
   Gly Leu Gln Arg Val Ala Val Gly Ala Ala Leu Leu Ser Met Pro Val
           35                  40                  45
   Arg Thr Gly Asp Thr Val Asn Asp Glu Asp Ile Ser Asn Thr Ile Arg
       50                  55                  60
   Ala Leu Phe Ala Thr Gly Asn Phe Glu Asp Val Arg Val Leu Arg Asp
   65                  70                  75                  80
   Gly Asp Thr Leu Leu Val Gln Val Lys Glu Arg Pro Thr Ile Ala Ser
                   85                  90                  95
   Ile Thr Phe Ser Gly Asn Lys Ser Val Lys Asp Asp Met Leu Lys Gln
               100                 105                 110
   Asn Leu Glu Ala Ser Gly Val Arg Val Gly Glu Ser Leu Asp Arg Thr
           115                 120                 125
   Thr Ile Ala Asp Ile Glu Lys Gly Leu Glu Asp Phe Tyr Tyr Ser Val
       130                 135                 140
   Gly Lys Tyr Ser Ala Ser Val Lys Ala Val Val Thr Pro Leu Pro Arg
   145                 150                 155                 160
   Asn Arg Val Asp Leu Lys Leu Val Phe Gln Glu Gly Val Ser Ala Glu
                   165                 170                 175
   Ile Gln Gln Ile Asn Ile Val Gly Asn His Ala Phe Thr Thr Asp Glu
               180                 185                 190
   Leu Ile Ser His Phe Gln Leu Arg Asp Glu Val Pro Trp Trp Asn Val
           195                 200                 205
   Val Gly Asp Arg Lys Tyr Gln Lys Gln Lys Leu Ala Gly Asp Leu Glu
       210                 215                 220
   Thr Leu Arg Ser Tyr Tyr Leu Asp Arg Gly Tyr Ala Arg Phe Asn Ile
   225                 230                 235                 240
   Asp Ser Thr Gln Val Ser Leu Thr Pro Asp Lys Lys Gly Ile Tyr Val
                   245                 250                 255
   Thr Val Asn Ile Thr Glu Gly Asp Gln Tyr Lys Leu Ser Gly Val Glu
               260                 265                 270
   Val Ser Gly Asn Leu Ala Gly His Ser Ala Glu Ile Glu Gln Leu Thr
           275                 280                 285
   Lys Ile Glu Pro Gly Glu Leu Tyr Asn Gly Thr Lys Val Thr Lys Met
       290                 295                 300
   Glu Asp Asp Ile Lys Lys Leu Leu Gly Arg Tyr Gly Tyr Ala Tyr Pro
   305                 310                 315                 320
   Arg Val Gln Ser Met Pro Glu Ile Asn Asp Ala Asp Lys Thr Val Lys
                   325                 330                 335
   Leu Arg Val Asn Val Asp Ala Gly Asn Arg Phe Tyr Val Arg Lys Ile
               340                 345                 350
   Arg Phe Glu Gly Asn Asp Thr Ser Lys Asp Ala Val Leu Arg Arg Glu
           355                 360                 365
   Met Arg Gln Met Glu Gly Ala Trp Leu Gly Ser Asp Leu Val Asp Gln
       370                 375                 380
   Gly Lys Glu Arg Leu Asn Arg Leu Gly Phe Phe Glu Thr Val Asp Thr
   385                 390                 395                 400
   Asp Thr Gln Arg Val Pro Gly Ser Pro Asp Gln Val Asp Val Val Tyr
                   405                 410                 415
   Lys Val Lys Glu Arg Asn Thr Gly Ser Phe Asn Phe Gly Ile Gly Tyr
               420                 425                 430
   Gly Thr Glu Ser Gly Val Ser Phe Gln Ala Gly Val Gln Gln Asp Asn
           435                 440                 445
   Trp Leu Gly Thr Gly Tyr Ala Val Gly Ile Asn Gly Thr Lys Asn Asp
       450                 455                 460
   Tyr Gln Thr Tyr Ala Glu Leu Ser Val Thr Asn Pro Tyr Phe Thr Val
   465                 470                 475                 480
   Asp Gly Val Ser Leu Gly Gly Arg Leu Phe Tyr Asn Asp Phe Gln Ala
                   485                 490                 495
   Asp Asp Ala Asp Leu Ser Asp Tyr Thr Asn Lys Ser Tyr Gly Thr Asp
               500                 505                 510
   Val Thr Leu Gly Phe Pro Ile Asn Glu Tyr Asn Ser Leu Arg Ala Gly
           515                 520                 525
   Leu Gly Tyr Val His Asn Ser Leu Ser Asn Met Gln Pro Gln Val Ala
       530                 535                 540
   Met Trp Arg Tyr Leu Tyr Ser Met Gly Glu His Pro Ser Thr Ser Asp
   545                 550                 555                 560
   Gln Asp Asn Ser Phe Lys Thr Asp Asp Phe Thr Phe Asn Tyr Gly Trp
                   565                 570                 575
   Thr Tyr Asn Lys Leu Asp Arg Gly Tyr Phe Pro Thr Asp Gly Ser Arg
               580                 585                 590
   Val Asn Leu Thr Gly Lys Val Thr Ile Pro Gly Ser Asp Asn Glu Tyr
           595                 600                 605
   Tyr Lys Val Thr Leu Asp Thr Ala Thr Tyr Val Pro Ile Asp Asp Asp
       610                 615                 620
   His Lys Trp Val Val Leu Gly Arg Thr Arg Trp Gly Tyr Gly Asp Gly
   625                 630                 635                 640
   Leu Gly Gly Lys Glu Met Pro Phe Tyr Glu Asn Phe Tyr Ala Gly Gly
                   645                 650                 655
   Ser Ser Thr Val Arg Gly Phe Gln Ser Asn Thr Ile Gly Pro Lys Ala
               660                 665                 670
   Val Tyr Phe Pro His Gln Ala Ser Asn Tyr Asp Pro Asp Tyr Asp Tyr
           675                 680                 685
   Glu Cys Ala Thr Gln Asp Gly Ala Lys Asp Leu Cys Lys Ser Asp Asp
       690                 695                 700
   Ala Val Gly Gly Asn Ala Met Ala Val Ala Ser Leu Glu Phe Ile Thr
   705                 710                 715                 720
   Pro Thr Pro Phe Ile Ser Asp Lys Tyr Ala Asn Ser Val Arg Thr Ser
                   725                 730                 735
   Phe Phe Trp Asp Met Gly Thr Val Trp Asp Thr Asn Trp Asp Ser Ser
               740                 745                 750
   Gln Tyr Ser Gly Tyr Pro Asp Tyr Ser Asp Pro Ser Asn Ile Arg Met
           755                 760                 765
   Ser Ala Gly Ile Ala Leu Gln Trp Met Ser Pro Leu Gly Pro Leu Val
       770                 775                 780
   Phe Ser Tyr Ala Gln Pro Phe Lys Lys Tyr Asp Gly Asp Lys Ala Glu
   785                 790                 795                 800
   Gln Phe Gln Phe Asn Ile Gly Lys Thr Trp
                   805                 810
<210> 5
   <211> 1491
   <212> DNA
   <213> Artificial Sequence
<220>
   <221> source
   <222> 1..1491
   <223> /mol_type="DNA" /note="CYP3A4 in plasmid pSC001" /organism="Artificial Sequence"
<400> 5
   ctcgagatgg ctctgttatt agcagttttt ctggtgctcc tctatctata tggaacccat      60
   tcacatggac tttttaagaa gcttggaatt ccagggccca cacctctgcc ttttttggga     120
   aatattttgt cctaccataa gggcttttgt atgtttgaca tggaatgtca taaaaagtat     180
   ggaaaagtgt ggggctttta tgatggtcaa cagcctgtgc tggctatcac agatcctgac     240
   atgatcaaaa cagtgctagt gaaagaatgt tattctgtct tcacaaaccg gaggcctttt     300
   ggtccagtgg gatttatgaa aagtgccatc tctatagctg aggatgaaga atggaagaga     360
   ttacgatcat tgctgtctcc aaccttcacc agtggaaaac tcaaggagat ggtccctatc     420
   attgcccagt atggagatgt gttggtgaga aatctgaggc gggaagcaga gacaggcaag     480
   cctgtcacct tgaaagacgt ctttggggcc tacagcatgg atgtgatcac tagcacatca     540
   tttggagtga acatcgactc tctcaacaat ccacaagacc cctttgtgga aaacaccaag     600
   aagcttttaa gatttgattt tttggatcca ttctttctct caataacagt ctttccattc     660
   ctcatcccaa ttcttgaagt attaaatatc tgtgtgtttc caagagaagt tacaaatttt     720
   ttaagaaaat ctgtaaaaag gatgaaagaa agtcgcctcg aagatacaca aaagcaccga     780
   gtggatttcc ttcagctgat gattgactct cagaattcaa aagaaactga gtcccacaaa     840
   gctctgtccg atctggagct cgtggcccaa tcaattatct ttatttttgc tggctatgaa     900
   accacgagca gtgttctctc cttcattatg tatgaactgg ccactcaccc tgatgtccag     960
   cagaaactgc aggaggaaat tgatgcagtt ttacccaata aggcaccacc cacctatgat    1020
   actgtgctac agatggagta tcttgacatg gtggtgaatg aaacgctcag attattccca    1080
   attgctatga gacttgagag ggtctgcaaa aaagatgttg agatcaatgg gatgttcatt    1140
   cccaaagggg tggtggtgat gattccaagc tatgctcttc accgtgaccc aaagtactgg    1200
   acagagcctg agaagttcct ccctgaaaga ttcagcaaga agaacaagga caacatagat    1260
   ccttacatat acacaccctt tggaagtgga cccagaaact gcattggcat gaggtttgct    1320
   ctcatgaaca tgaaacttgc tctaatcaga gtccttcaga acttctcctt caaaccttgt    1380
   aaagaaacac agatccccct gaaattaagc ttaggaggac ttcttcaacc agaaaaaccc     1440
   gttgttctaa aggttgagtc aagggatggc accgtaagtg gagccggtac c             1491
<210> 6
   <211> 497
   <212> PRT
   <213> Artificial Sequence
<220>
   <221> SOURCE
   <222> 1..497
   <223> /mo1_type="protein" /note="Amino acid sequence of CYP3A4 in plasmid pSC001" /organism="Artificial Sequence"
<400> 6
   Leu Glu Met Ala Leu Leu Leu Ala Val Phe Leu Val Leu Leu Tyr Leu
   1               5                   10                  15
   Tyr Gly Thr His Ser His Gly Leu Phe Lys Lys Leu Gly Ile Pro Gly
               20                  25                  30
   Pro Thr Pro Leu Pro Phe Leu Gly Asn Ile Leu Ser Tyr His Lys Gly
           35                  40                  45
   Phe Cys Met Phe Asp Met Glu Cys His Lys Lys Tyr Gly Lys Val Trp
       50                  55                  60
   Gly Phe Tyr Asp Gly Gln Gln Pro Val Leu Ala Ile Thr Asp Pro Asp
   65                  70                  75                  80
   Met Ile Lys Thr Val Leu Val Lys Glu Cys Tyr Ser Val Phe Thr Asn
                   85                  90                  95
   Arg Arg Pro Phe Gly Pro Val Gly Phe Met Lys Ser Ala Ile Ser Ile
               100                 105                 110
   Ala Glu Asp Glu Glu Trp Lys Arg Leu Arg Ser Leu Leu Ser Pro Thr
           115                 120                 125
   Phe Thr Ser Gly Lys Leu Lys Glu Met Val Pro Ile Ile Ala Gln Tyr
       130                 135                 140
   Gly Asp Val Leu Val Arg Asn Leu Arg Arg Glu Ala Glu Thr Gly Lys
   145                 150                 155                 160
   Pro Val Thr Leu Lys Asp Val Phe Gly Ala Tyr Ser Met Asp Val Ile
                   165                 170                 175
   Thr Ser Thr Ser Phe Gly Val Asn Ile Asp Ser Leu Asn Asn Pro Gln
               180                 185                 190
   Asp Pro Phe Val Glu Asn Thr Lys Lys Leu Leu Arg Phe Asp Phe Leu
           195                 200                 205
   Asp Pro Phe Phe Leu Ser Ile Thr Val Phe Pro Phe Leu Ile Pro Ile
       210                 215                 220
   Leu Glu Val Leu Asn Ile Cys Val Phe Pro Arg Glu Val Thr Asn Phe
   225                 230                 235                 240
   Leu Arg Lys Ser Val Lys Arg Met Lys Glu Ser Arg Leu Glu Asp Thr
                   245                 250                 255
   Gln Lys His Arg Val Asp Phe Leu Gln Leu Met Ile Asp Ser Gln Asn
               260                 265                 270
   Ser Lys Glu Thr Glu Ser His Lys Ala Leu Ser Asp Leu Glu Leu Val
           275                 280                 285
   Ala Gln Ser Ile Ile Phe Ile Phe Ala Gly Tyr Glu Thr Thr Ser Ser
       290                 295                 300
   Val Leu Ser Phe Ile Met Tyr Glu Leu Ala Thr His Pro Asp Val Gln
   305                 310                 315                 320
   Gln Lys Leu Gln Glu Glu Ile Asp Ala Val Leu Pro Asn Lys Ala Pro
                   325                 330                 335
   Pro Thr Tyr Asp Thr Val Leu Gln Met Glu Tyr Leu Asp Met Val Val
               340                 345                 350
   Asn Glu Thr Leu Arg Leu Phe Pro Ile Ala Met Arg Leu Glu Arg Val
           355                 360                 365
   Cys Lys Lys Asp Val Glu Ile Asn Gly Met Phe Ile Pro Lys Gly Val
       370                 375                 380
   Val Val Met Ile Pro Ser Tyr Ala Leu His Arg Asp Pro Lys Tyr Trp
   385                 390                 395                 400
   Thr Glu Pro Glu Lys Phe Leu Pro Glu Arg Phe Ser Lys Lys Asn Lys
                   405                 410                 415
   Asp Asn Ile Asp Pro Tyr Ile Tyr Thr Pro Phe Gly Ser Gly Pro Arg
               420                 425                 430
   Asn Cys Ile Gly Met Arg Phe Ala Leu Met Asn Met Lys Leu Ala Leu
           435                 440                 445
   Ile Arg Val Leu Gln Asn Phe Ser Phe Lys Pro Cys Lys Glu Thr Gln
       450                 455                 460
   Ile Pro Leu Lys Leu Ser Leu Gly Gly Leu Leu Gln Pro Glu Lys Pro
   465                 470                 475                 480
   Val Val Leu Lys Val Glu Ser Arg Asp Gly Thr Val Ser Gly Ala Gly
                   485                 490                 495
   Thr
<210> 7
   <211> 1482
   <212> DNA
   <213> Escherichia coli
<220>
   <221> source
   <222> 1..1482
   <223> /mol_type="DNA"
<400> 7
   atgaagaaat tgctccccat tcttatcggc ctgagccttt ctgggttcag ttcgttgagc     60
   caggccgaga acctgatgca agtttatcag caagcacgcc ttagtaaccc ggaattgcgt    120
   aagtctgccg ccgatcgtga tgctgccttt gaaaaaatta atgaagcgcg cagtccatta    180
   ctgccacagc taggtttagg tgcagattac acctatagca acggctaccg cgacgcgaac    240
   ggcatcaact ctaacgcgac cagtgcgtcc ctgcagttaa ctcaatccat ttttgatatg    300
   tcgaaatggc gtgcgttaac gctgcaggaa aaagcagcag ggattcagga cgtcacgtat    360
   cagaccgatc agcaaacctt gatcctcaac accgcgaccg cttatttcaa cgtgttgaat    420
   gctattgacg ttctttccta tacacaggca caaaaagaag cgatctaccg tcaattagat    480
   caaaccaccc aacgttttaa cgtgggcctg gtagcgatca ccgacgtgca gaacgcccgc    540
   gcacagtacg ataccgtgct ggcgaacgaa gtgaccgcac gtaataacct tgataacgcg    600
   gtagagcagc tgcgccagat caccggtaac tactatccgg aactggctgc gctgaatgtc    660
   gaaaacttta aaaccgacaa accacagccg gttaacgcgc tgctgaaaga agccgaaaaa    720
   cgcaacctgt cgctgttaca ggcacgcttg agccaggacc tggcgcgcga gcaaattcgc    780
   caggcgcagg atggtcactt accgactctg gatttaacgg cttctaccgg gatttctgac    840
   acctcttata gcggttcgaa aacccgtggt gccgctggta cccagtatga cgatagcaat    900
   atgggccaga acaaagttgg cctgagcttc tcgctgccga tttatcaggg cggaatggtt    960
   aactcgcagg tgaaacaggc acagtacaac tttgtcggtg ccagcgagca actggaaagt   1020
   gcccatcgta gcgtcgtgca gaccgtgcgt tcctccttca acaacattaa tgcatctatc   1080
   agtagcatta acgcctacaa acaagccgta gtttccgctc aaagctcatt agacgcgatg   1140
   gaagcgggct actcggtcgg tacgcgtacc attgttgatg tgttggatgc gaccaccacg   1200
   ttgtacaacg ccaagcaaga gctggcgaat gcgcgttata actacctgat taatcagctg   1260
   aatattaagt cagctctggg tacgttgaac gagcaggatc tgctggcact gaacaatgcg   1320
   ctgagcaaac cggtttccac taatccggaa aacgttgcac cgcaaacgcc ggaacagaat   1380
   gctattgctg atggttatgc gcctgatagc ccggcaccag tcgttcagca aacatccgca   1440
   cgcactacca ccagtaacgg tcataaccct ttccgtaact ga                       1482
<210> 8
   <211> 493
   <212> PRT
   <213> Escherichia coli
<220>
   <221> SOURCE
   <222> 1..493
   <223> /mol_type="protein" /note="Amino acid sequence of the tolC gene of E.coli"
<400> 8
   Met Lys Lys Leu Leu Pro Ile Leu Ile Gly Leu Ser Leu Ser Gly Phe
   1               5                   10                  15
   Ser Ser Leu Ser Gln Ala Glu Asn Leu Met Gln Val Tyr Gln Gln Ala
               20                  25                  30
   Arg Leu Ser Asn Pro Glu Leu Arg Lys Ser Ala Ala Asp Arg Asp Ala
           35                  40                  45
   Ala Phe Glu Lys Ile Asn Glu Ala Arg Ser Pro Leu Leu Pro Gln Leu
       50                  55                  60
   Gly Leu Gly Ala Asp Tyr Thr Tyr Ser Asn Gly Tyr Arg Asp Ala Asn
   65                  70                  75                  80
   Gly Ile Asn Ser Asn Ala Thr Ser Ala Ser Leu Gln Leu Thr Gln Ser
                   85                  90                  95
   Ile Phe Asp Met Ser Lys Trp Arg Ala Leu Thr Leu Gln Glu Lys Ala
               100                 105                 110
   Ala Gly Ile Gln Asp Val Thr Tyr Gln Thr Asp Gln Gln Thr Leu Ile
           115                 120                 125
   Leu Asn Thr Ala Thr Ala Tyr Phe Asn Val Leu Asn Ala Ile Asp Val
       130                 135                 140
   Leu Ser Tyr Thr Gln Ala Gln Lys Glu Ala Ile Tyr Arg Gln Leu Asp
   145                 150                 155                 160
   Gln Thr Thr Gln Arg Phe Asn Val Gly Leu Val Ala Ile Thr Asp Val
                   165                 170                 175
   Gln Asn Ala Arg Ala Gln Tyr Asp Thr Val Leu Ala Asn Glu Val Thr
               180                 185                 190
   Ala Arg Asn Asn Leu Asp Asn Ala Val Glu Gln Leu Arg Gln Ile Thr
           195                 200                 205
   Gly Asn Tyr Tyr Pro Glu Leu Ala Ala Leu Asn Val Glu Asn Phe Lys
       210                 215                 220
   Thr Asp Lys Pro Gln Pro Val Asn Ala Leu Leu Lys Glu Ala Glu Lys
   225                 230                 235                 240
   Arg Asn Leu Ser Leu Leu Gln Ala Arg Leu Ser Gln Asp Leu Ala Arg
                   245                 250                 255
   Glu Gln Ile Arg Gln Ala Gln Asp Gly His Leu Pro Thr Leu Asp Leu
               260                 265                 270
   Thr Ala Ser Thr Gly Ile Ser Asp Thr Ser Tyr Ser Gly Ser Lys Thr
           275                 280                 285
   Arg Gly Ala Ala Gly Thr Gln Tyr Asp Asp Ser Asn Met Gly Gln Asn
       290                 295                 300
   Lys Val Gly Leu Ser Phe Ser Leu Pro Ile Tyr Gln Gly Gly Met Val
   305                 310                 315                 320
   Asn Ser Gln Val Lys Gln Ala Gln Tyr Asn Phe Val Gly Ala Ser Glu
                   325                 330                 335
   Gln Leu Glu Ser Ala His Arg Ser Val Val Gln Thr Val Arg Ser Ser
               340                 345                 350
   Phe Asn Asn Ile Asn Ala Ser Ile Ser Ser Ile Asn Ala Tyr Lys Gln
           355                 360                 365
   Ala Val Val Ser Ala Gln Ser Ser Leu Asp Ala Met Glu Ala Gly Tyr
       370                 375                 380
   Ser Val Gly Thr Arg Thr Ile Val Asp Val Leu Asp Ala Thr Thr Thr
   385                 390                 395                 400
   Leu Tyr Asn Ala Lys Gln Glu Leu Ala Asn Ala Arg Tyr Asn Tyr Leu
                   405                 410                 415
   Ile Asn Gln Leu Asn Ile Lys Ser Ala Leu Gly Thr Leu Asn Glu Gln
               420                 425                 430
   Asp Leu Leu Ala Leu Asn Asn Ala Leu Ser Lys Pro Val Ser Thr Asn
           435                 440                 445
   Pro Glu Asn Val Ala Pro Gln Thr Pro Glu Gln Asn Ala Ile Ala Asp
       450                 455                 460
   Gly Tyr Ala Pro Asp Ser Pro Ala Pro Val Val Gln Gln Thr Ser Ala
   465                 470                 475                 480
   Arg Thr Thr Thr Ser Asn Gly His Asn Pro Phe Arg Asn
                   485                 490
<210> 9
   <211> 93
   <212> DNA
   <213> Artificial Sequence
<220>
   <221> source
   <222> 1..93
   <223> /mol_type="DNA" /note="Fragment of a typical artificial autotransporter" /organism="Artificial Sequence"
<400> 9
   ctatcttcag catatgcaca tggaacacct tctagactcg agagatcttg ccctgaatat     60
   ttcaaaggtc caccttctcc acgatctctt aat                                  93
<210> 10
   <211> 31
   <212> PRT
   <213> Artificial Sequence
<220>
   <221> SOURCE
   <222> 1..31
   <223> /mol_type="protein" /note="Fragment of a typical artificial autotransporter" /organism="Artificial Sequence"
<400> 10
   Leu Ser Ser Ala Tyr Ala His Gly Thr Pro Ser Arg Leu Glu Arg Ser
   1               5                   10                  15
   Cys Pro Glu Tyr Phe Lys Gly Pro Pro Ser Pro Arg Ser Leu Asn
               20                  25                  30
<210> 11
   <211> 24
   <212> DNA
   <213> Artificial Sequence
<220>
   <221> source
   <222> 1..24
   <223> /mol_type="DNA" /note="Fragment with important restriction site " /organism="Artificial Sequence"
<400> 11
   actgatttgc tcgagatgga agaa 24
<210> 12
   <211> 8
   <212> PRT
   <213> Artificial Sequence
<220>
   <221> SOURCE
   <222> 1..8
   <223> /mol_type="protein" /note="Fragment with important restriction site" /organism="Artificial Sequence"
<400> 12
   Thr Asp Leu Leu Glu Met Glu Glu
   1 5
<210> 13
   <211> 24
   <212> DNA
   <213> Artificial Sequence
<220>
   <221> source
   <222> 1..24
   <223> /mol_type="DNA" /note="Fragment with important restriction site" /organism="Artificial Sequence"
<400> 13
   agccgcatgg gtacccttaa tcct 24
<210> 14
   <211> 8
   <212> PRT
   <213> Artificial Sequence
<220>
   <221> SOURCE
   <222> 1..8
   <223> /mol_type="protein" /note="Fragment with important restriction site" /organism="Artificial Sequence"
<400> 14
   Ser Arg Met Gly Thr Leu Asn Pro
   1 5